(19) Europäisches Patentamt
European Patent Office
Office européen des brevets

(11) Publication number: **0 341 961 A2**

(12) **EUROPEAN PATENT APPLICATION**

(21) Application number: 89304645.8

(22) Date of filing: 08.05.89

(51) Int. Cl.4: C07C 121/50 , C07C 101/20 , C07C 103/84 , C07C 101/447 , C07C 79/38 , C07C 69/612 , C07C 69/767 , C07J 1/00 , C07D 333/34 , A61K 31/215 , A61K 31/275

(30) Priority: 09.05.88 US 192034

(43) Date of publication of application: 15.11.89 Bulletin 89/46

(84) Designated Contracting States: AT BE CH DE ES FR GB IT LI LU NL SE

(71) Applicant: MERCK & CO. INC.
126, East Lincoln Avenue P.O. Box 2000
Rahway New Jersey 07065-0900(US)

(72) Inventor: Thompson, Wayne J.
Box 97 Whites Mill Road
Green Lane, PA 18054(US)

Inventor: Thompson, David D.
2149 Old Forde Way
Lansdale, PA 19446(US)
Inventor: Anderson, Paul S.
1233 Buttonwood Drive
Lansdale, PA 19446(US)
Inventor: Rodan, Gideon A.
827 Deerfield Lane
Bryn Mawr, PA 19010(US)

(74) Representative: Hesketh, Alan, Dr.
European Patent Department Merck & Co., Inc. Terlings Park Eastwick Road
Harlow Essex, CM20 2QR(GB)

(54) **Polymalonic acids as boneaffinity agents.**

(57) The invention relates to novel polymalonic acids which act as bone-affinity agents. Compounds of the formula

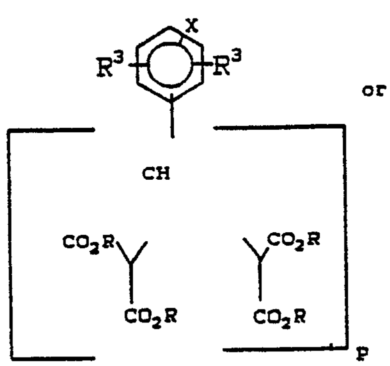

or

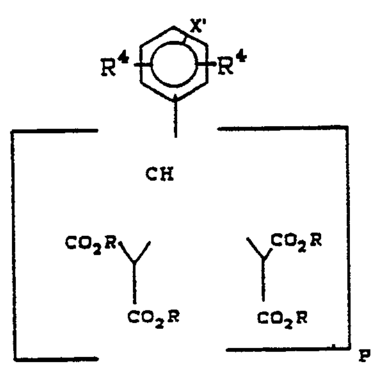

have a high affinity for bone and are useful in the treatment and prevention of diseases involving bone

EP 0 341 961 A2

resorption, particularly osteoporosis, Paget's disease, and malignant hypercalcemia, by delivering a bone resorption or formation active-drug directly to the bone target site.

# POLYMALONIC ACIDS AS BONE-AFFINITY AGENTS

## BACKGROUND OF THE INVENTION

The present invention relates to novel polymalonic acid compounds, processes for their preparation, pharmaceutical compositions containing them, and methods for their use as bone-affinity agents for delivering bone resorption or formation active drugs directly to the bone target site.

Hitherto, it was known that certain compounds exhibit a bone-seeking affinity. In this context, bone-seeking affinity relates to the ability of the compound to bind to mineralized bone matrix with a tendancy to accumulate in bone and to incorporate into the crystalline apatite structure. Tetracyclines and diphosphonates such as, for example, 4-amino-1-hydroxy-butane-1,1-diphosphonic acid, 3-amino-1-hydroxy-propane-1,1-diphosphonic acid, ethane-1-hydroxy-1,1-diphosphonic acid and dichloromethane diphosphonic acid, are representative compounds known to have bone-seeking affinity.

Additionally, it has been proposed to join a bone-seeking agent, such as tetracyclines or diphosphonates, to a carbonic anhydrase inhibitor through a bridging agent to provide compounds for the treatment or prophylaxis of degenerative bone diseases. EP 201,057 (published November 12, 1986).

The present invention is based on discoveries related to the binding site for the Gla protein (osteocalcin). The three α-carboxylated glutamic acid residues and one arginine of osteocalcin can assume a planar configuration with atomic distances identical with interatomic distances between three calcium ions and one phosphate radical on the hydroxylapatite crystalline lattice surface. The polymalonates of the present invention fit this configuration and thus their bone affinity properties result from attachment directly to the bone target site.

## DESCRIPTION OF THE INVENTION

The compounds of the present invention are best characterized by reference to the following structural formula I:

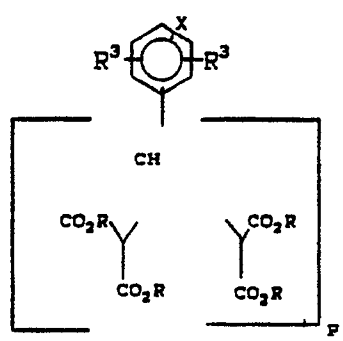

wherein:

X is

(1) hydrogen;

(2) alkyl having 1 to 6 carbon atoms

(3) $-(CH_2)_n(R^2)_2$ M

wherein n is 0 to 6 and M is

a) halogen;

b) $-NR^1R^1$;

c) $-NHC(O)R^1$;

d) $-NO_2$;

e) $-C(O)R^1$;

f) $-C(O)OR^1$;

g) -CN;

each $R^1$ is independently H, $C_1$ to $C_6$ straight or branched alkyl, $(CH_2)_m$ OH where m is 1 to 3, benzyl or

substituted benzyl wherein the substituents are $C_1$ to $C_6$ alkyl, halogen, CN or $CF_3$, phenyl or substituted phenyl wherein the substituents are $C_1$ to $C_6$ alkyl, halogen, CN or $CF_3$;
$R^2$ is absent or is H, $C_1$ to $C_6$ straight or branched alkyl, $C_3$ to $C_6$ cycloalkyl, $(CH_2)_m$ OH where m is 1 to 3;
$R^3$ is H, $C_1$ to $C_6$ straight or branched alkyl, $(CH_2)_m$ OH where m is 1 to 3;
R is H, $C_1$ to $C_6$ alkyl, benzyl or a pharmaceutically acceptable salt; and
p is 1 or 2.

The embodiment of the invention represented as formula I are polymalonic acids which themselves are bone-affinity agents and may be useful as therapeutic agents. These compounds attach directly to bone and accumulate therein.

In another embodiment of the invention, the bone-affinity properties of the polymalonic acid compounds of formula I can be advantageously used as a drug delivery agents. In this embodiment, the polymalonic acids are covalently linked to drugs which modulate bone resorption or formation by a linker substituent. This allows delivery of the entire molecular entity to the bone target site of the drug. At the bone target site, the linker substituent is cleaved by enzymatic action and the bone resorption or formation active drug is released. Use of the polymalonic acids as drug delivery agents results in use of reduced amounts of the bone resorption or formation active-drugs, thus lowering toxicity or other unwanted side-effects related to these drugs.

The compounds of the alternative embodiment of the invention are best characterized by reference to the following structural formula II:

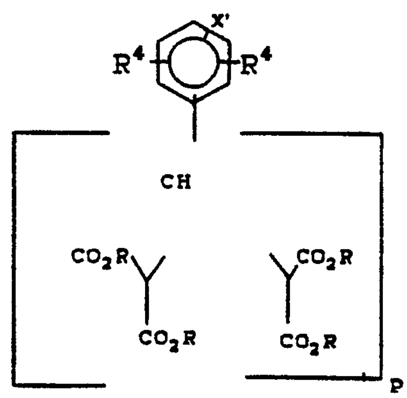

wherein:
$X'$ is $-(CH_2)_n (R^5)_2$ M
wherein n is 0 to 6 and M is
a) $-NHC(O)R^7$;
b) $-OC(O)R^7$;
c)

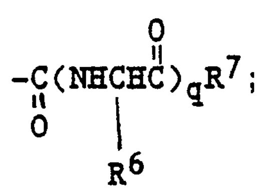

$R^5$ is absent or is H, C, $C_1$ to $C_6$ straight or branched alkyl, $C_3$ to $C_6$ cycloalkyl, $(CH_2)_m$ OH where m is 1 to 3;
$R^4$ is H, $C_1$ to $C_6$ straight or branched alkyl, $(CH_2)_m$ OH where m is 1 to 3;
$R^6$ is a natural amino acid side chain;
R is H, $C_1$ to $C_6$ alkyl, benzyl or a pharmaceutically acceptably salt;
p is 1 or 2; and
q is 0 to 3;
$R^7$ is a deprotonated bone resorption inhibitor or bone formation promoter.

PREFERRED EMBODIMENTS OF THE INVENTION

The preferred embodiments of the instant invention are realized in structural formula III:

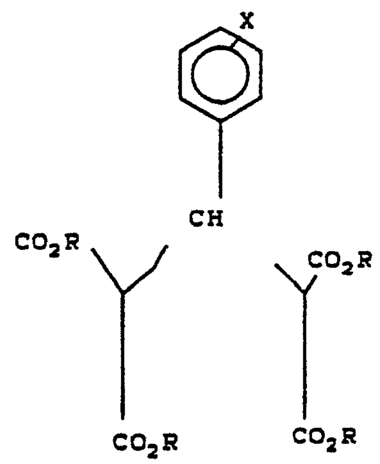

wherein:
X is $NH_2$, $C_1$ to $C_6$ alkylamine or $NO_2$; and
R is hydrogen, $C_1$ to $C_6$ alkyl, benzyl or a pharmaceutically acceptable salt.

Preferred compounds of the invention of formula I are:
tetraethyl(4-cyanobenzylidene)dimalonate;
tetraethyl(4-aminomethylbenzylidene)dimalonate;
tetraethyl-N-benzoyl(4-aminomethylbenzylidene)dimalonate;
tetraethyl-N-4-iodobenzoyl(4-aminomethylbenzylidene)dimalonate;
tetralithium-N-benzoyl(4-aminomethylbenzylidene)dimalonate;
tetralithium-N-4-iodobenzoyl(4-aminomethylbenzylidene)dimalonate;
1,3-bis[tetraethyl(2-propane-1,1,3,3-tetracarboxylate)]-5-nitrobenzene;
3,5-bis[tetraethyl(2-propane-1,1,3,3-tetracarboxylate)]aniline;
tetraethyl(4-nitrobenzylidene)dimalonate;
tetraethyl(4-aminobenzylidene)dimalonate;
tetraethyl(4-benzyloxycarbonyl)benzylidene dimalonate;
tetraethyl 4-carboxybenzylidene dimalonate;
tetrabenzyl(4-cyanobenzylidene)dimalonate;
tetrabenzyl(4-aminomethylbenzylidene)dimalonate;

Preferred compounds of the alternative embodiment of the invention of formula II are:
N-[methyl-4-phenyl(tetraethyl-1,1,3,3-propane tetracarboxylate)-0-methyl-4-phenylsulfonyl(5-thiophene-2-sulfonamide)]-carbamate;
N-[carbonyl-4-benzene(tetraethyl-2-propane-1,1,3,3-tetracarboxylate]-5-(3-acetoxybenzoyl)thiophene-2-sulfonamide;
N-[carbonyl-4-benzene(tetralithio-2-propane-1,1,3,3-tetracarboxylate]-5-(3-acetoxybenzoyl)thiophene-2-sulfonamide;
N-[N-α-[carbonyl-4-benzene(tetraethyl-2-propane-1,1,3,3-tetracarboxylate)]-L-leucyl-5-(3-hydroxybenzoyl)-thiophene-2-sulfonamide;
N-[N-α-[carbonyl-4-benzene(tetralithio-2-propane-1,1,3,3-tetracarboxylate)]-L-leucyl]-5-(3-hydroxybenzoyl)-thiophene-2-sulfonamide.
Estra-1,3,5(10)-triene-3,17-beta-diol-17-[N-(methyl-4-phenyl(tetrasodium-2-propane-1,1,3,3-tetracarboxylate)-)carbamate];
Estra-6,7-dideuterio-1,3,5(10)-triene-3-17-beta-diol-17-[N-(methyl-4-phenyl(tetrasodium-2-propane-1,1,3,3-tetracarboxylate))carbamate];
Estra-1,3,5(10)-triene-3,17-beta-diol[N-(methyl-4-phenyl(tetrasodium-2-propane-1,1,3,3-tetracarboxylate)-)carbamate].

The drugs or agents which modulate bone resorption or stimulate bone formation that can be used in the present invention are any of a wide variety of classes. These drugs are covalently linked through a linker substituent, preferrably an alkylamine linker substituent, to the polymalonic acids of formula I. The

drugs can be active as bone resorption inhibitors such as carbonic anhydrase inhibitors, such as for example 5-[(4-hydroxymethylphenyl)sulfonyl]thiophene-2-sulfonamide, 5-(3-hydroxybenzoyl)thiophene-2-sulfonamide, 5-(3-acetoxybenzoyl)thiophene-2-sulfonamide, proteinase inhibitors, such as for example collagenase inhibitors, cysteine proteinase inhibitors, serine proteinase inhibitors, cathepsin inhibitors, such as L-trans-epoxysuccinyl-L-leuccylamido(e-guanidino)butane (E-64), lysosomal enzyme inhibitors, ion channel blockers, ion transport blockers, cytoskeleton disruptive agents, calcitonin, calcitonin mimicking molecules, or bone formation promoters, such as prostaglandins, for example prostaglandin $E_2$, and bone growth factors, such as transforming growth factor in all of its forms, particularly TGF-$\beta$, insulin-like growth factor in all of its forms, particularly IGF-1 and IGF-2, acid or basic fibroblast growth factor in all of its forms, particularly or aFGF and bFGF, and interleukin such as IL-3. Additionally, drugs which act as both bone resorption inhibiting and bone formation stimulating agents such as bone active steroids can be effectively used in the present invention. For example, estrogens including synthetic steroidal and nosteroidal compounds with estrogenic activity , natural steroidal estrogens, particularly estrodial, and all of their conjugates, androgens including synthetic steroidal and nonsteroidal compounds with androgenic activity, natural steroidal androgens, and all of their conjugates, and glucocorticoid inhibitors are useful in the present invention.

Included within the scope of this invention are all the enantiomers of any compound of the invention which exhibits optical isomerism. Additionally, all pharmaceutically acceptable salts of the compounds described herein, such as sodium, potassium, lithium, ammonium and the like, salts are also within the scope of this invention. Also included within the scope of the invention are the analogs and derivatives of the parent polymalonic acids, their salts, esters, amides and other derivatives which may be prepared by conventional methods well known to those of ordinary skill in the art. "Halogen" as utilized herein means chlorine, fluorine, bromine and iodine. The natural amino acid side chain in formula II is a single amino acid selected from the common, naturally occuring amino acid, such as A, R, N, D, C, Q, E, G, H, I, L, K, M, F, P, S, T, W, Y and V.

The synthesis of the compounds of formula I and II are generally carried out by the following route. It will be readily apparent to one of ordinary skill in the art reviewing the synthetic routes depicted below that other compounds within formula I and II can be synthesized by substitution of appropriate reactants and agents in the synthesis shown below. Michael condensation method. The bis-malonate tetra-alkyl ester is then purified in the usual manner and the amino carboxyl linking functionality is formed by hydrogenolysis or metal hydride reduction. The amino-functionalized bis-malonate tetra-alkyl ester is coupled to the drug by condensing with phosgene or diacid chloride. The carboxy-functionalized bis-malonate tetra-alkyl ester is converted to active ester or acid chloride in the usual manner used for forming esters or amides and then coupled with the desired drug.

In the final step, the active bis-malonate tetracarboxylate is liberated from the tetra-ester by saponification or the more preferred technique of hydrogenolysis of the tetra-benzyl ester. The tetra-carboxylate salt is isolated and purified by recrystallization from water by addition of the appropriate solvent.

## Scheme I

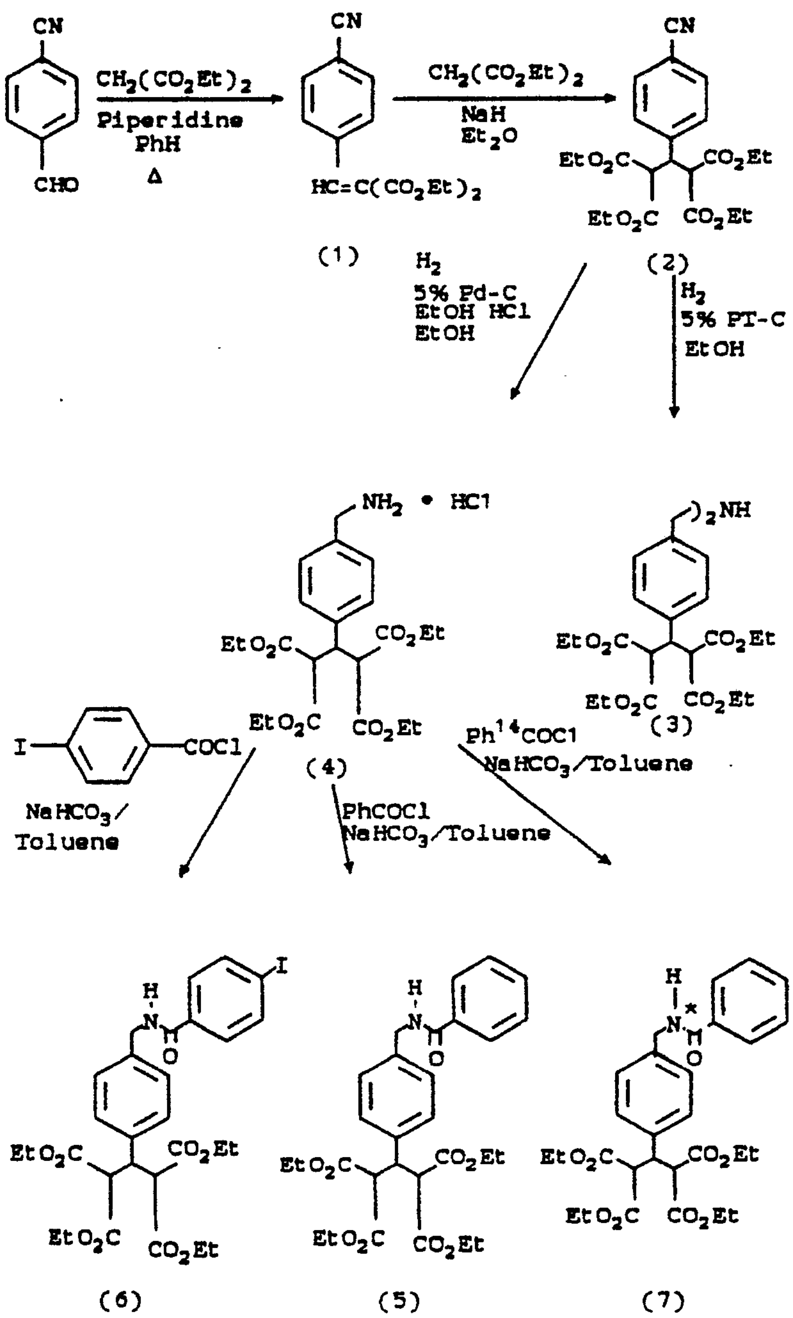
$CH_2(CO_2Et)_2$
Piperidine
PhH
Δ
$CH_2(CO_2Et)_2$
NaH
$Et_2O$
(1)
(2)
$H_2$
5% Pd-C
EtOH HCl
EtOH
$H_2$
5% PT-C
EtOH
(4)
(3)
NaHCO$_3$/
Toluene
PhCOCl
NaHCO$_3$/Toluene
$Ph^{14}COCl$
NaHCO$_3$/Toluene
(6)
(5)
(7)

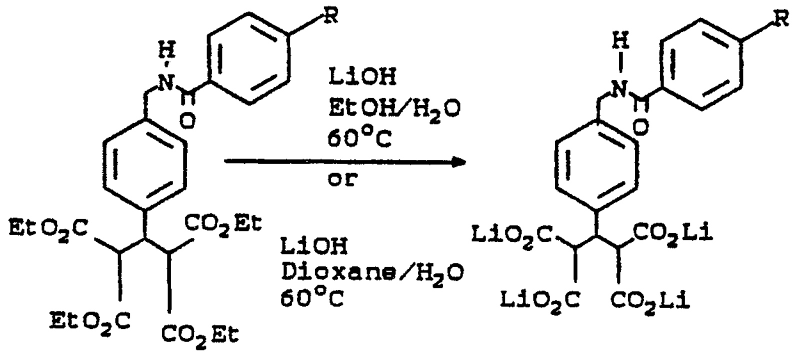


R=H or I
(5.6)

R=H or I
(8.9)

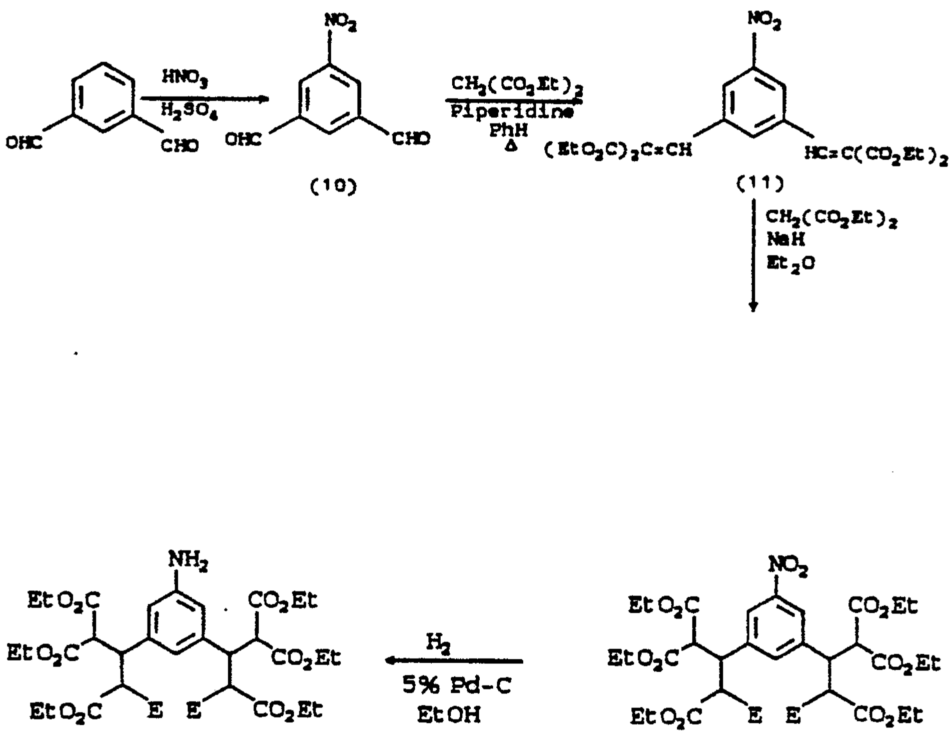


(10)

(11)

13

12

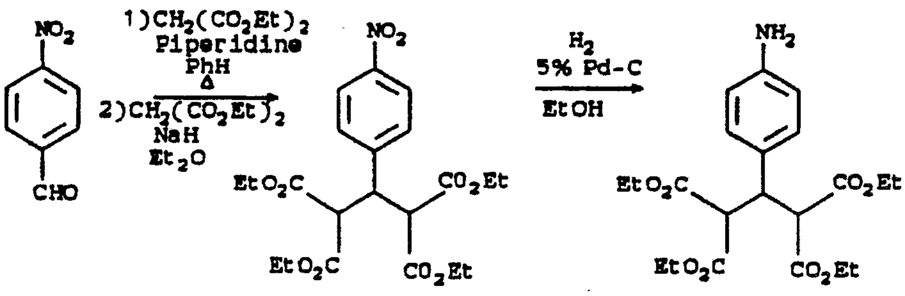


(14) (15)

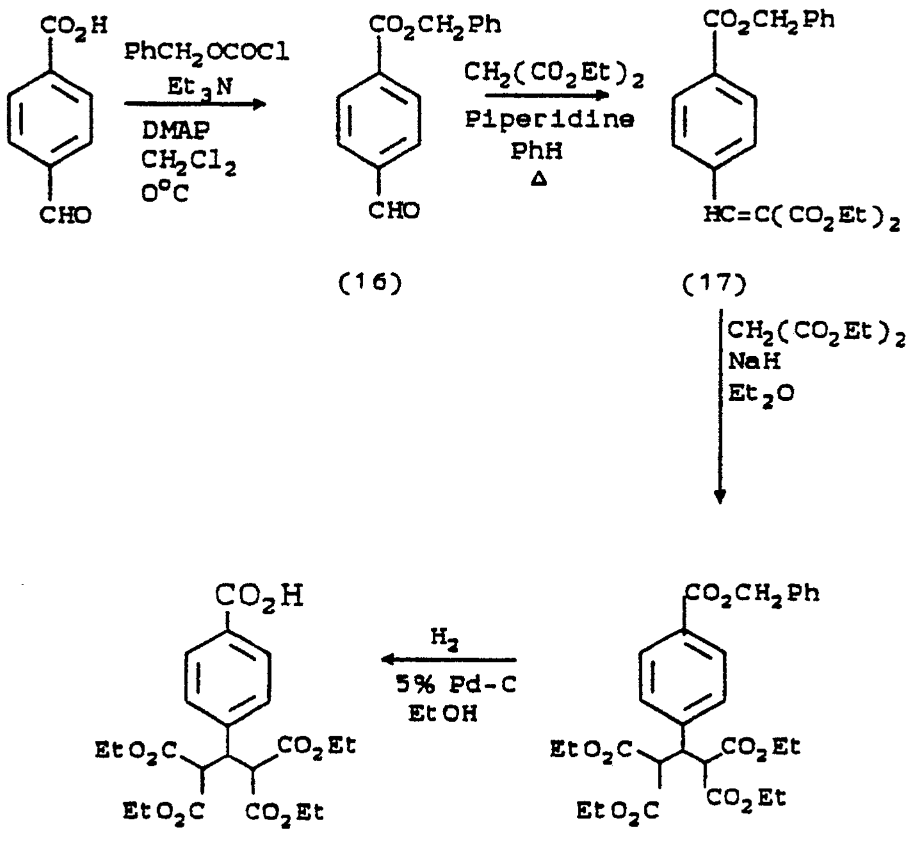


(16) (17)

(19) (18)

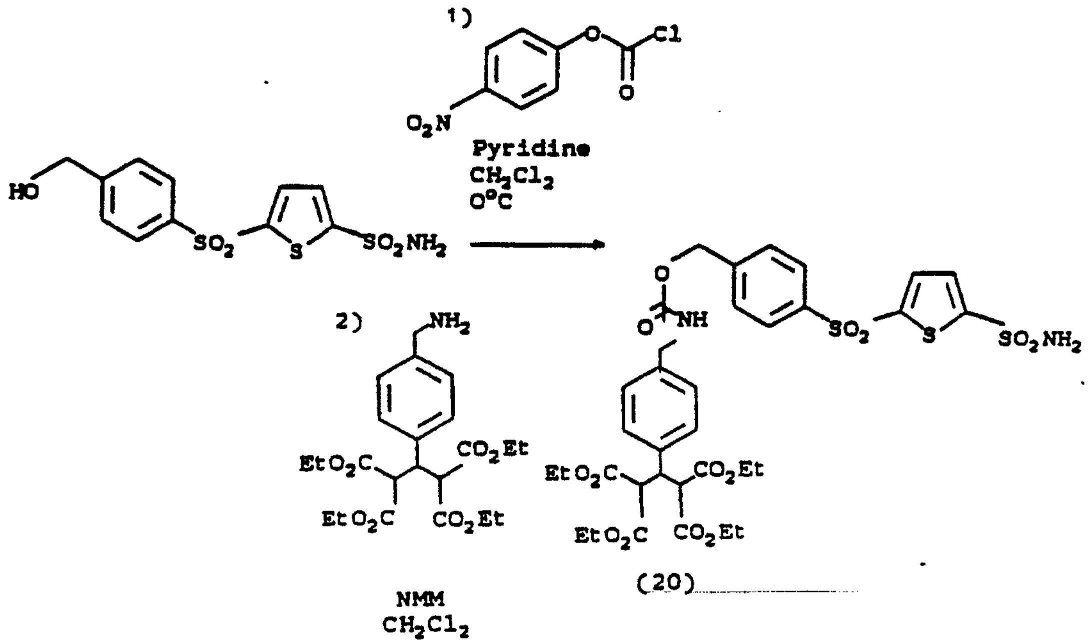
1)
Pyridine
$CH_2Cl_2$
0°C
2)
NMM
$CH_2Cl_2$

(20)

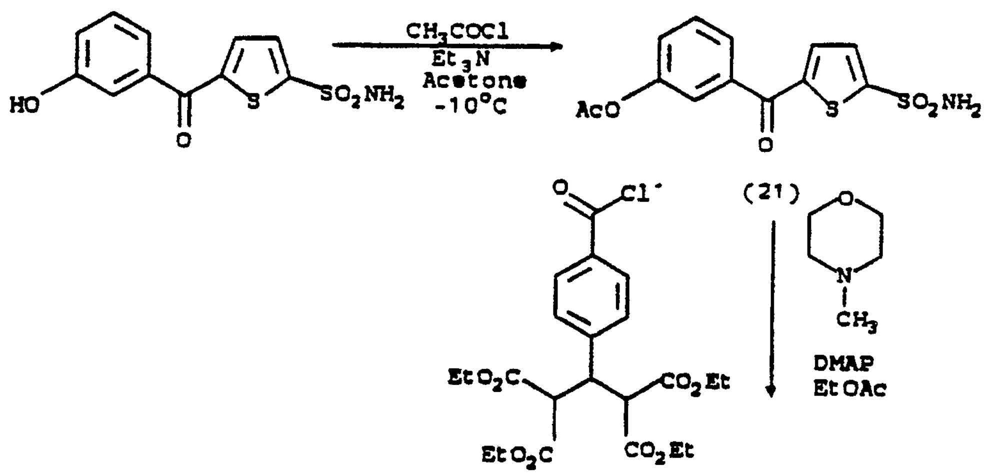
$CH_3COCl$
$Et_3N$
Acetone
-10°C
DMAP
EtOAc

(21)

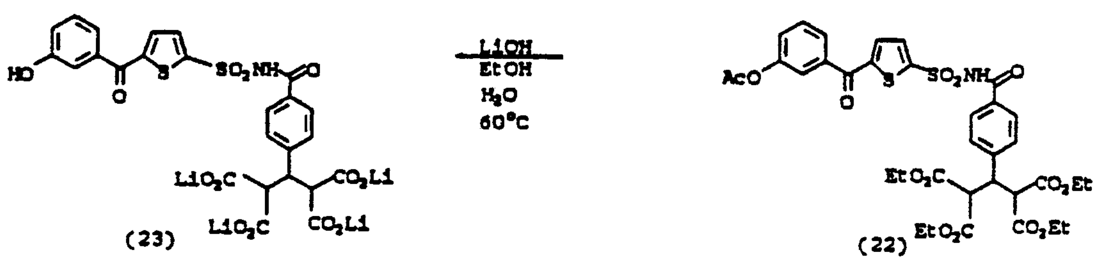
LiOH
EtOH
$H_2O$
60°C

(23)

(22)

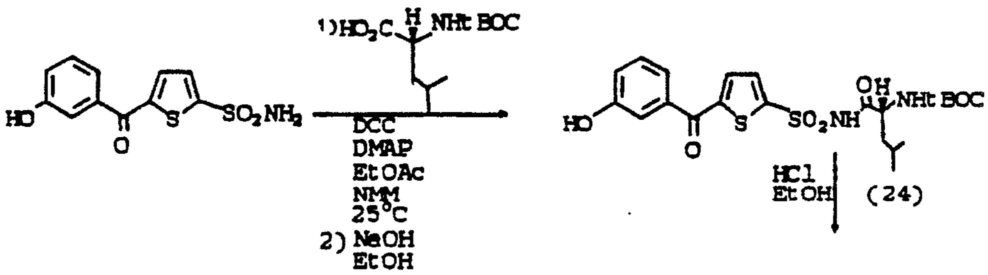
1) $HO_2C$ NHt BOC
DCC
DMAP
EtOAc
NMM
25°C
2) NaOH
EtOH
HCl
EtOH

(24)

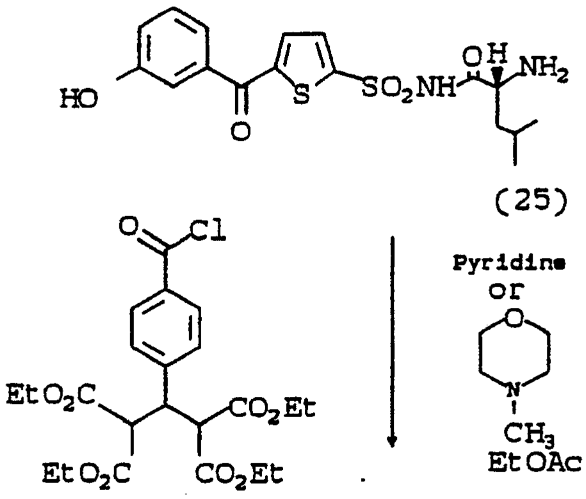
Pyridine
or
EtOAc

(25)

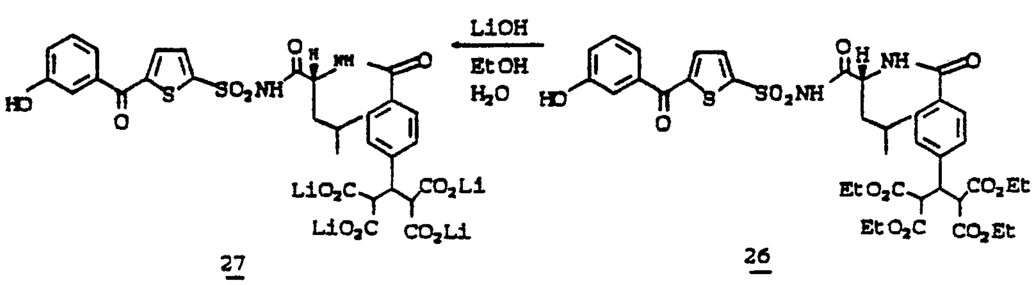
LiOH
EtOH
$H_2O$

27

26

# SCHEME II

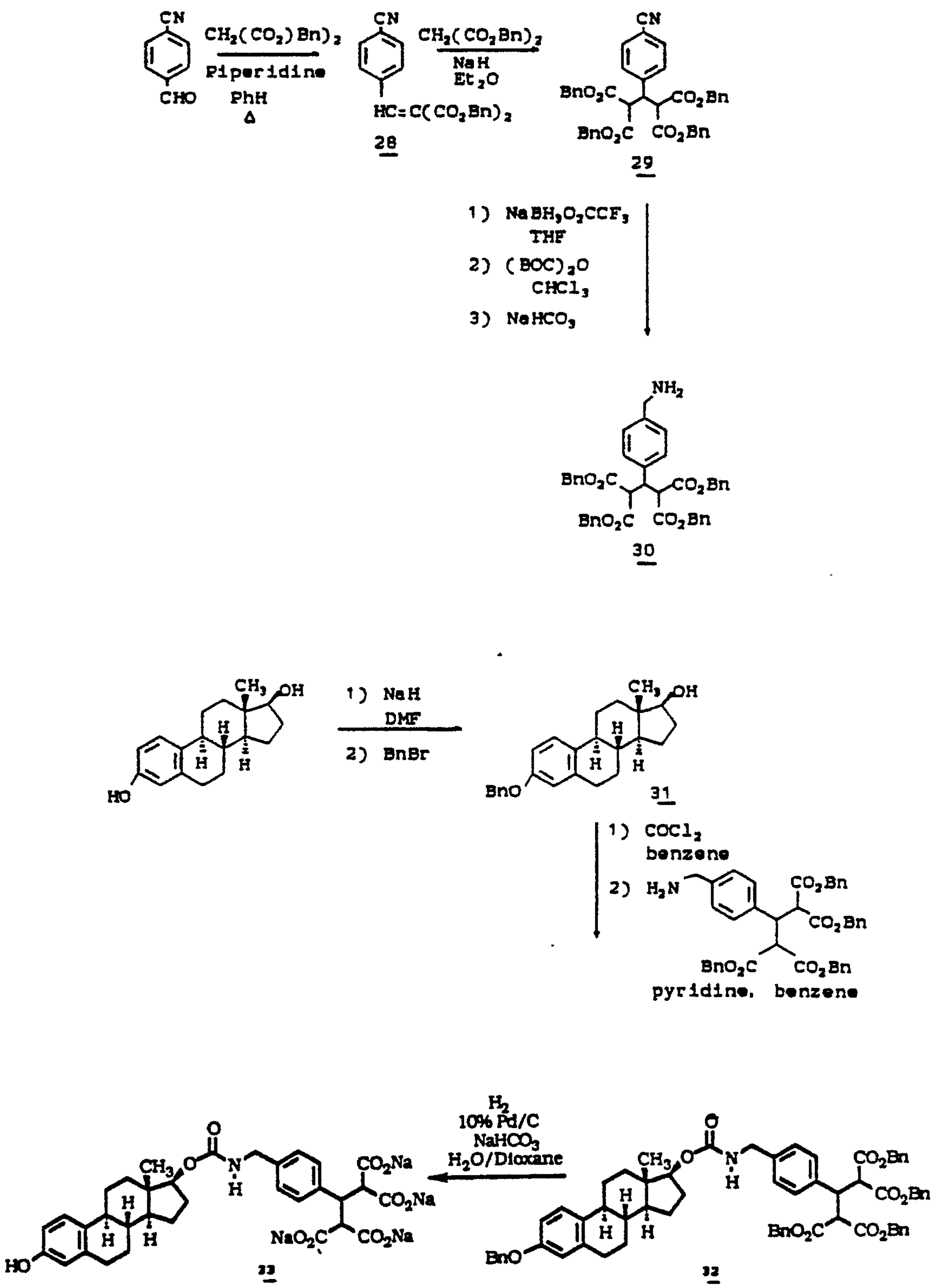

$CH_2(CO_2)Bn)_2$
Piperidine
PhH
Δ
28
$CH_2(CO_2Bn)_2$
NaH
$Et_2O$
29
1) $NaBH_3O_2CCF_3$ THF
2) $(BOC)_2O$ $CHCl_3$
3) $NaHCO_3$
30
1) NaH DMF
2) BnBr
31
1) $COCl_2$ benzene
2)
pyridine, benzene
$H_2$
10% Pd/C
$NaHCO_3$
$H_2O$/Dioxane
33
32

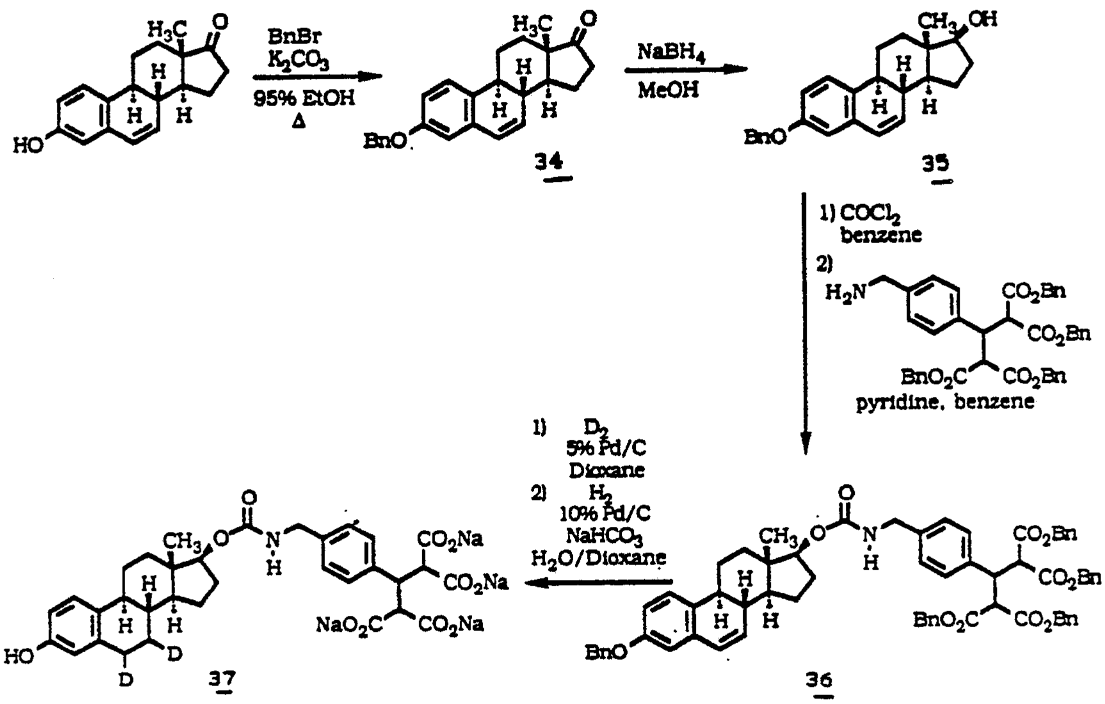


34 35 36 37

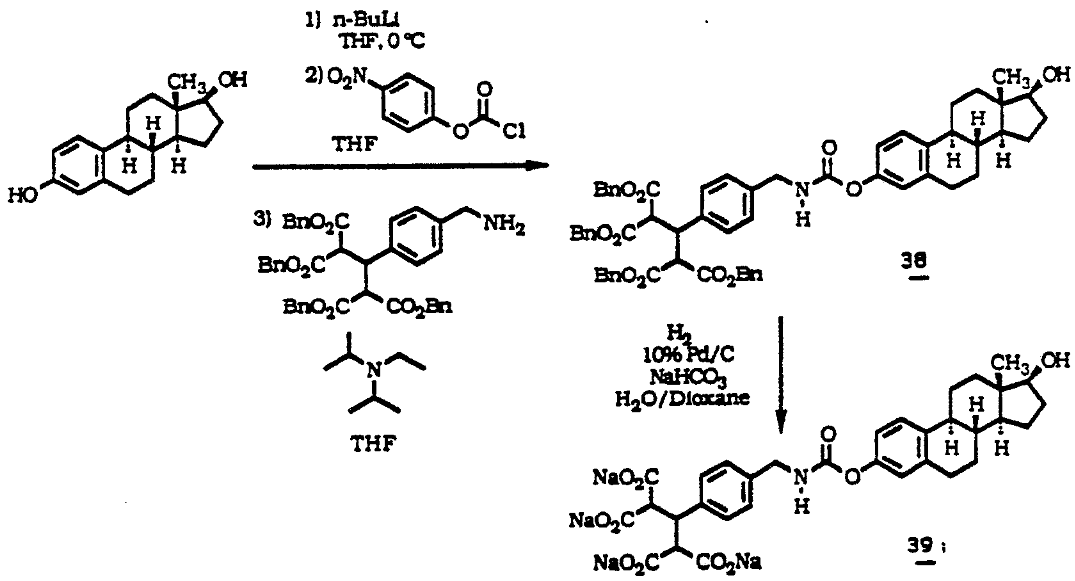


38

39 ;

The magnitude of a prophylactic or therapeutic dose of a compound of formula I or II will, of course, vary with the nature or the severity of the condition to be treated and with the particular compound of formula I or II and its route of administration. In general, the daily dose range for bone resorption disease use lies within the range of from about .01 mg to about 10 mg per kg body weight of a mammal.

Any suitable route of administration may be employed for providing a mammal, especially a human with an effective dosage of a compound of formula I or II. For example, oral, rectal, topical, parenteral, ocular, nasal, buccal, intravenous and the like may be employed. Dosage forms include tablets, troches, dispersions, suspensions, solutions, capsules, creams, ointments, aerosols and the like.

The pharmaceutical compositions of the present invention comprise a compound of formula I or II as an active ingredient or a pharmaceutically acceptable salt thereof, and may also contain a pharmaceutically acceptable carrier and optionally other therapeutic ingredients. The term "pharmaceutically acceptable

salts" refers to salts prepared from pharmaceutically acceptable non-toxic acids or bases including inorganic and organic acids and bases.The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well-known in the art of pharmacy.

For administration by inhalation, the compounds of the present invention are conveniently delivered in the form of an aerosol spray from pressurized packs or a nebuliser, or a powder which may be formulated as a cartridge from which the powder composition may be inhaled with the aid of a suitable device. The preferred delivery system for inhalation in a metered dose inhalation (MDI) aerosol, which may be formulated as a suspension or solution in fluorocarbon propellants.

Suitable topical formulations of compounds of formula I or II include transdermal devices, aerosols, creams, ointments, lotions, dusting powder, and the like.

In practical use, a compound of formula I or II can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the compounds of formula I or II may also be administered by controlled release means and/or delivery devices.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent.

The following are examples of representative pharmaceutical dosage forms for the compounds of formula I or II:

| Injectable Suspension (I.M.) | mg/ml |
|---|---|
| Compound of Example 23 | 2.0 |
| Methylcellulose | 5.0 |
| Tween 80 | 0.5 |
| Benzyl alcohol | 9.0 |
| Benzalkonium chloride | 1.0 |
| Water for injection to a total volume of 1 ml | |

| Tablet | mg/tablet |
|---|---|
| Compound of Example 23 | 25.0 |
| Microcrystalline Cellulose | 415.0 |
| Providone | 14.0 |
| Pregelatinized Starch | 43.5 |
| Magnesium Stearate | 2.5 |
| | 500 |

| Capsule | mg/capsule |
|---|---|
| Compound of Example 23 | 25.0 |
| Lactose Powder | 573.5 |
| Magnesium Stearate | 1.5 |
| | 600 |

## EXAMPLE 1

Preparation of 1, Diethyl(4-cyanobenzylidene)malonate:

A mixture of 4-cyanobenzaldehyde (50.1 g, 380 mmol), diethyl malonate (70 ml, 460 mmol), piperidene (4.2 ml, 42 mmol) and benzene (300 ml) was heated under vigorous reflux with azeotropic removal of water for 69 hr. The reaction mixture was concentrated in vacuo to give a yellow oil which was dissolved in ethyl acetate (300 ml), washed with 1N HCl(1x100 ml), satd. $NaHCO_3$ (1x100 ml) and satd. NaCl(1x100 ml), dried over anhyd. $Na_2SO_4$, filtered, and concentrated to give a yellow oil. Crude product was triturated with diethyl ether/hexanes to give a white solid which was collected by vacuum filtration and air dried to yield 87.9 g (84%) diethyl (4-cyanobenzylidene) malonate: $^1$H-NMR (300MHz, $CDCl_3$) δ 1.30 (t, 3H, J = 7Hz), 1.35 (t, 3H, J - 7Hz), 4.32 (q, 4H, J = 7.2Hz), 7.56 (d, 2H, J = 8.1Hz), 7.67 (d,2H,J=6.9Hz), 7.71 (s,1H).

## EXAMPLE 2

Preparation of 2, Tetraethyl(4-cyanobenzylidene)dimalonate:

To a mechanically stirred suspension of sodium hydride (450 mmol, from 17.93 g 60% in oil dispersion, rinsed twice with dry hexanes) in 550 ml dry diethyl ether under nitrogen was added dropwise a solution of diethyl malonate (62 ml, 408 mmol) in 250 ml dry diethyl ether. The resultant thick suspension was stirred at RT for 20 min then a solution of 4-cyanobenzal ethyl malonate (93.22 g, 341 mmol) in 1.0 1 dry diethyl ether was added in a fine stream. The reaction mixture was stirred at RT for 4 hr then poured into 1N HCl (500 ml). The organic phase was collected and the aqueous solution was extracted with of ethyl acetate (3x200 ml). The combined organics were washed with satd. NaCl (1x200 ml), dried over anhyd. $Na_2SO_4$, filtered, and concentrated to give a yellow oil. Oil was triturated with hexanes to give a fine white solid which was collected by vacuum filtration, and air dried to yield 140.8 g (95%) tetraethyl (4-cyanobenzylidene) dimalonate: $^1$H-NMR (300MHz, $CDCl_3$ δ 1.04 (t, 6H, J = 14.7Hz, J = 6.9Hz), 1.22 (t, 6H, J = 6.9Hz), 4.00 (1, 4H, J = 14.7Hz, J = 6.9 Hz), 4.15 (q, 4H, J = 14.7Hz, J = 6.3Hz), 7.51 (d, 2H, J = 8.7Hz), 7.56 (d, 2H, J = 8.7Hz).

## EXAMPLE 3

Preparation of 3, Octaethyl[(4-benzylidenedimalonate)methyl]amine:

A mixture of tetraethyl (4-cyanobenzylidene)dimalonate (3.03 g, 7.0 mmol) and 5% platinum on carbon catalyst (1.53 g) in 40 ml abs. ethanol was shaken under $H_2$(50 psi) in a Parr® apparatus for 112 hr then filtered through a pad of Celite®. Filtrate was concentrated in vacuo to give 2.7 g octaethyl[(4-benzylidenedimalonate)methyl]amine (90%) which was purified by low pressure chromatography (silica gel, 230-400 mesh; ethyl acetate(50%): hexanes(50%): $^1$H-NMR(300MHz, $CDCl_3$)ð 1.03 (t, 6H), 1.2 (t, 6H), 1.52 (br s, 1H), 3.69 (s, 1H), 3.393 (q, 4H), 4.05-4.25 (m, 7H), 7.16 (d, 2H, J = 8Hz), 7.30(d, 2H, J = 8Hz).

EXAMPLE 4

Preparation of 4, Tetraethyl(4-aminomethylbenzylidene)dimalonate:

A mixture of tetraethyl (4-cyanobenzylidene)dimalonate (20.3 g, 47 mmol), 5% palladium on carbon catalyst (5.0 g), and ethanolic HCl (6.95 N, 22 ml, 153 mmol) in 70 ml abs. ethanol was shaken under $H_2$ - (50 psi) in a Parr® appratus for 72 hr then filtered through a pad of Celite®. Filtrate was concentrated in vacuo to give 22.1 g (quant. yield) tetraethyl (4-aminomethylbenzylidene) dimalonate as the hydrochloride salt: $^1$H-NMR (300 MHz, $CDCl_3$) ð: 1.06 (t, 6H, J = 7.1Hz), 1.24 (t,6H, J = 7.1Hz), 3.95 (q, 4H, J = 7.1Hz), 4.0-4.2 (complex mult., 7H), 7.33 (d, 2H, J = 8.0Hz), 7.43 (d, 2H, J = 8Hz), 8.45 (br s, 2H).

EXAMPLE 5

Preparation of 5, Tetraethyl-N-benzoyl(4-aminomethylbenzylidene)dimalonate:

To a suspension of tetraethyl (4-aminomethylbenzylidene)dimalonate (10.85 g, 23 mmol) in toluene (50 ml) was added satd. $NaHCO_3$ (50 ml) followed by benzoyl chloride (2.8 ml, 24 mmol). The reaction mixture was stirred vigorously at RT for 5 hr and the phases were allowed to separate. The organic phase was collected and the aqueous phase was extracted with diethyl ether (3x25 ml). The combined organics were washed with satd. $NaHCO_3$ (2x25 ml), 1N HCl (1x25 ml), and satd. NaCl (1x25 ml), dried over anhyd. $MgSO_4$, filtered, and concentrated to give 12.77 g white solid. The crude product was purified by recrystallization twice from diethyl ether/hexanes to yield 10.63 g (86%) tetraethyl-N-benzoyl(4-aminomethylbenzylidene)dimalonate as a fine white solid: m.p. 87-88.5 °C; $^1$H-NMR (300MHz, $CDCl_3$) ð 1.03 (t,6H, J = 7.1Hz), 1.23 (t,6H, J = 7.2Hz), 3.96 (q, 4H, J = 7.1Hz), 4.05-4.25 (complex mult., 7H), 4.59 (d, 2H, J = 5.6Hz), 6.37 (br s, 1H), 7.24 (d, 2H, J = 8Hz), 7.45 (d, 2H, J = 8Hz), 7.40-7.55 (complex mult., 1H), 7.45 (d, 2H, J = 8Hz), 7.77 (d, 2H, J = 7.5Hz); elemental analysis, calcd. for $C_{29}H_{35}NO_9$ (541.60): C = 64.31, H = 6.51, N = 2.59; found: C = 64.39, H = 6.48, N = 2.64.

EXAMPLE 6

Preparation of 6, Tetraethyl-N-4 iodobenzoyl(4-aminomethylbenzylidene)dimalonate:

To a suspension of tetraethyl(4-aminomethylbenzylidene)dimalonate (10.00 g, 21 mmol) in toluene (50 ml) was added satd. $NaHCO_3$ (50 ml) followed by 4-iodobenzoyl chloride (5.90g, 22 mmol). The reaction mixture was stirred vigorously at RT for 18 hr and the phases were allowed to separate. The organic phase was collected and the aqueous phase was extracted with diethyl ether (3x25 ml). The combined organics were washed with satd. $NaHCO_3$ (2x25 ml), 1 N HCl (1x25 ml), and satd. NaCl (1x25 ml), dried over anhyd. $MgSO_4$, filtered, and concentrated to give 14.6 g clear oil. The crude product was purified by recrystallization twice from diethyl ether/hexanes to yield 13.07 g (93%) tetraethyl-N-4-iodoboenzoyl (4-aminomethylbenzylidene)dimalonate as a fine white solid: m.p. 85-86.5 °C; $^1$H-NMR (300MHz, $CDCl_3$) ð

1.03 (t, 6H, J = 7Hz), 1.23 (t, 6H, J = 7Hz), 3.95 (q, 4H, J = 7Hz), 4.05-4.25 (complex mult., 7H), 4.55 (d, 2H, J = 6Hz), 6.39 (br s, 1H), 7.21 (d, 2H, J = 7Hz), 7.34 (d, 2H, J = 7Hz), 7.50 (d, 2H, J = 8.Hz), 7.79 (d, 2H, J = 8.Hz); elemental analysis, calcd. for $C_{29}H_{34}INO_9$ (667.49): C=52.18, H=5.13, N=2.10; found: C=51.85, H=5.14, N=2.09.

## EXAMPLE 7

Preparation of 7, Tetraethyl-N-$^{14}$C-carbonylbenzoyl(4-aminomethylbenzylidene)dimalonate:

To a vigorously stirred solution of tetraethyl (4-aminomethylbenzylidene)dimalonate (380 mg, 0.87 mmol) in toluene (1.5 ml) and satd. $NHCO_3$ (2.0 ml) was added a solution of benzoyl chloride carbonyl-$^{14}$C (5.0 mCi, 0.46 mmol, 10.9 mCi/mmol) in toluene (2.0 ml, 2.5 mCi/ml), (ampule rinsed with 1.0 ml toluene). Reaction was stirred at RT for 1 hr then was extracted with diethyl ether (4x4 ml). Organics were washed with satd. $NaHCO_3$ (3x3 ml), 1N HCl (4x3 ml), and satd. NaCl (1x3 ml), dried over anhyd. $Na_2SO_4$, filtered and concentrated under a stream of nitrogen to give a light yellow oily residue which was dissolved in diethyl ether (90%): ethyl acetate (10%) and filtered through a pad of silica gel (70-230 mesh) in a 30 ml fine frit (pad rinsed with diethyl ether (90%): ethyl acetate (10%). Concentration of filtrate under a stream of nitrogen gave a light green oil which was triturated with petroleum ether; solvent was removed under a stream of nitrogen with scratching to give a gummy solid which was triturated with diethyl ether to give a fine white solid. Solid was collected by vacuum filtration and air dried to yield 101 mg (41%) tetraethyl-N-$^{14}$C-carbonylbenzoyl (4-aminomethylbenzylidene) dimalonate: 97.5% radiochemical purity by TLC (silica gel; ethyl acetate (60%):hexanes (40%), specific activity= 17.29 µCi/mg.

## EXAMPLE 8

Preparation of 8, Tetralithium-N-benzoyl(4-aminomethylbenzylidene)dimalonate:

To a solution of tetraethyl-N-benzoyl(4-aminomethylbenzylidene)dimalonate (0.54813 g, 1.01 mmol) in 40 ml abs. ethanol was added 40 ml water followed by anhyd. lithium hydroxide (0.2918 g, 12.2 mmol), Reaction mixture was heated at 60° C for 24 hr with stirring then was concentrated in vacuo. Residue was dissolved in water and the solution was adjusted on pH 7.0 by the addition of 1N HCl. The solution was filtered through a medium frit and the volume was adjusted to 25 ml with water. Reverse phase HPLC (SpectraPhysics system, Waters Bondapack C18 column, flow rate = 1ml/min, $H_2O$;$CH_3CN$ gradient [98%:2% to 50%:50%]) indicated purity of solution to be 88% (@ 254 nm).

## EXAMPLE 9

Preparation of 9, Tetralithium-N-4-iodobenzoyl(4-aminomethylbenzylidene)dimalonate:

To a solution of tetraethyl-N-4-iodobenzoyl(4-aminomethylbenzylidene)dimalonate (0.713 g, 107 mmol) in a 40 ml dioxane was added 40 ml water follwed by anhyd. lithium hydroxide (0.307 g, 12.8 mmol). Reaction mixture was heated at 60° C for 20 hr with stirring then was triturated with 30 ml dioxane to give a suspension of a fine white solid. Solid was collected by vacuum filtration, air dried, and dried in vacuo (RT @ 0.1 torr) to yield 0.684 g (quant. yield) tetralithium-N-4-iodobenzoyl (4-aminomethylbenzylidene)-dimalonate: m.p. >300° C; $^1$H-NMR (300MHz, $D_2O$) ∂ 3.64 (d, 2H, J = 9Hz), 3.87 (t, 1H, J = 9Hz), 4.54 (s, 2H), 7.22 (d, 2H, J = 8Hz), 7.37 (d, 2H, J = 8Hz), 7.54 (d, 2H, J = 8.4Hz), 7.92 (d, 2H, J = 8.5Hz); elemental analysis, calcd. for $C_{21}H_{14}1Li_4NO_9$ • 1.5 $H_2O$ (606.03): C=41.62, H=2.83, N=2.31; found: C=41.57, H=3.11, N=1.99.

## EXAMPLE 10

### Preparation of 10, 5-Nitroisophthalaldehyde:

In a manner identical to that described by K.F. Jennings, J. Chem. Soc., 1172 (1957) red fuming nitric acid (43 ml, 1.0 mol) was added dropwise to a mechanically stirred mixture of ammonium sulfate (59 g, 446 mol) and conc. sulfuric acid (100 ml) cooled to 0°C in an ice bath such that the temperature remained below 10°C. To the chilled reaction mixture was added dropwise over a period of 40 min a solution of isophthalaldehyde (14.72 g, 110 mmol) in conc. sulfuric acid (100 ml) The reaction mixture was stirred at 5°C for 3 hr and at RT for 33 hr then poured into 2 kg crushed ice to five a fine light yellow solid which was collected by vacuum filtration and air dried to yield 14.53 g. The aqueous filtrate was extracted with ethyl acetate (3x400 ml); organics were washed with water (2x300 ml), dried over anhyd. $Na_2SO_4$, filtered, and concentrated to give 2.53 g tan solid. Both samples of crude product were combined and purified by low pressure chromatography (silica gel, 230-400 mesh; ethyl acetate (50%):hexanes (50%) to yield 15.13 g (83%) 5-nitroisophthalaldehyde: $^1H$-NMR (300MHz, $CDCl_3$) $\delta$ 8.73 (s, 1H), 8.96 (s,2H).

## EXAMPLE 11

### Preparation of 11, 1,3-bis[Diethyl(ethene-2,2-dicarboxylate)]-5-nitrobenzene:

A mixture of 5-nitroisophthalaldehyde (1.07 g, 6.0 mmol), diethyl malonate (1.96 ml, 13 mmol), piperidine (0.13 ml, 1.3 mmol), benzoic acid (0.03 g, 0.3 mmol), and benzene (20 ml) was heated under vigorous reflux with azeotropic removal of water for 17 hr. The reaction mixture was concentrated in vacuo to give a light orange solid which was dissolved in ethyl acetate (100 ml), washed with 1N HCl (2.50 ml) satd. $NaHCO_3$ (2.50 ml), and satd. NaCl(1.50 ml), dried over anhyd. $Na_2SO_4$, filtered, and concentrated to give an orange oil. Oil was triturated with diethyl ether/hexanes to give a white solid which was collected by vacuum filtration, air dried, and dried in vacuo to yield 1.86 g (67%) 1,3-bis[diethyl(ethene-2,2-dicarboxylate)]-5-nitrobenzene: m.p. 93-94.5°C; $^1H$-NMR (300MHz, $CDCl_3$) $\delta$ 1.32 (t, 6H, J = 6Hz), 1.37 (t, 6H, J = 6Hz), 4.36 (overlapping quartets, 8H, J = 6Hz, $\Delta\nu$ ~10Hz), 7.70 (s, 2H), 7.78 (s, 1H), 8.34 (s, 2H); elemental analysis, calcd. for $C_{22}H_{25}NO_{10}$ (463.44): C=57.02, H=5.44, N=3.02; found: C=57.24, H=5.30, N=3.12.

## EXAMPLE 12

### Preparation of 12, 1,3-bis[Tetraethyl(2-propane-1,1,3,3-tetracarboxylate)]-5-nitrobenzene:

To a stirred suspension of sodium hydride (8.9 mmol, from 0.36 g 60% in oil dispersion, rinsed thrice with dry hexanes) in 30 ml dry diethyl ether under nitrogen was added dropwise a solution of diethyl malonate (1.06 ml, 7.0 mmol) in 10 ml dry diethyl ether. To the resultant thick suspension was added in a fine stream a solution of 1,3-bis[diethyl(ethene-2,2-dicarboxylate]-5-nitrobenzene (0.80 g, 1.7 mmol) in 30 ml dry diethyl ether. The resultant yellow suspension was stirred at RT for 1.5 hr then poured into 50 ml 1N HCl. The organic phase was collected and the aqueous phase was extracted with ethyl acetate 3x50 ml). The combined organics were washed with satd. NaCl (1x50 ml), dried over anhyd. $Na_2SO_4$, filtered, and concentrated to give 1.57 g light yellow oil. Crude product was purified by low pressure chromatography (silica gel 230-400 mesh; hexanes (50%): diethyl ether (50%) to yield 1.17 g (86%) 1,3-bis[tetraethyl(2-propane-1,1,3,3-tetracarboxylate)]-5-nitrobenzene as a white solid: $^1H$-NMR (300MHz, $CDCl_3$) $\delta$ 1.11 (t, 12H, J = 6Hz), 1.25 (t, 12H, J = 6Hz), 4.01(q, 8H, J = 6Hz), 4.10-4.32 (complex mult., 14H), 7.83 (s, 1H), 8.12 (s, 2H).

## EXAMPLE 13

Preparation of 13, 3,5-bis[Tetraethyl(2-propane-1,1,3,3-tetracarboxylate)]aniline:

A mixture of 1,3-bis[tetraethyl(2-propane-1,1,3,3-tetracarboxylate)]-5-nitrobenzene (1.08 g, 1.4 mmol) and 5% palladium on carbon catalyst (0.41 g) in 50 ml abs. ethanol was shaken under $H_2$ (50 psi) in Parr® apparatus for 5 hr then filtered through a pad of Celite®. Filtrate was concentrated in vacuo to give 1.19 g (quant. yield) 3,5-bis[tetraethyl(2-propane-1,1,3,3-tetracarboxylate)]aniline as a clear oil: $^1$H-NMR (300MHz, $CDCl_3$) ∂ 1.11 (t, 12H, J = 6Hz), 1.25 (t, 12H, J = 6Hz), 3.59 (br s, 2H), 4.01(q, 8H, J = 6Hz), 4.05-4.20 (complex mult., 14H), 6.50 (s, 2H), 6.62 (s, 1H).

EXAMPLE 14

Preparation of 14, Tetraethyl(4-nitrobenzylidene)dimalonate:

In a manner similar to that described by Pratt and Werlbe, J. Am. Chem. Soc., 72, 4638-41 (1950), a mixture of 10 g 4-nitrobenzaldehyde, 15 g ethyl malonate, 20 ml of toluene, and 0.5 ml of piperidine were heated to reflux for 60 hrs under a Dean-Stark water separator. The mixture was cooled, diluted with 100 ml of ethyl acetate, washed with water, 1N HCl, and satd. $NaHCO_3$, dried over anhyd. $MgSO_4$, filtered, and concentrated. Upon trituration with diethyl ether and hexane tan colored crystals formed (4.4 g). Upon concentration another crop of product (2.1 g) was obtained. Both crops which were homogeneous by TLC were combined affording 6.5 g of diethyl(4-nitrobenzylidene)dimalonate: $^1$H-NMR (300MHz, $CDCl_3$) ∂ 1.30 (t,3H, J = 7.5Hz), 1.35 (t, 3H, J = 7Hz), 4.35 (overlapping quartets, 4H, J = 7Hz, Δv ~1Hz), 7.28 (s, 1H), 7.6 (d, 2H, J = 9Hz), 7.75 (s, 1H), 8.25 (d, 2H, J = 9Hz).

A solution of 2.9 g of diethyl(4-nitrobenzylidene)dimalonate in 100 ml of diethyl ether was added dropwise to a suspension of 2 equivalents of the sodium salt of diethyl malonate from 3.3 g of diethyl malonate and 1 g of sodium hydride (60% oil dispersion) in 50 ml of diethyl ether. After stirring for 1 hr, the mixture was poured into 100 ml of 1N HCl and extracted with 200 ml of ethyl acetate. The organic layer was dried over anhyd. $MgSO_4$, filtered, and concentrated. Low pressure chromatography (silica gel 230-400 mesh; diethyl ether (25%):hexanes (75%)) gave 4.0 g tetraethyl(4-nitrobenzylidene)dimalonate after drying at 80°C, 0.01 mm Hg for 2 hr. $^1$H-NMR (300MHz, $CDCl_3$) ∂ 1.1 (t, 6H, J = 7Hz), 1.25 (t, 6H, J = 7Hz), 4.0 (q, 4H, J = 7Hz), 4.18 (q, 4H, J = 7Hz), 4.18 (overlapping doublet, 2H, J = 8Hz), 4.35 (t, 1H, J = 8Hz), 7.6 (d, 2H, J = 9Hz), 8.15 (d, 2H, J = 9Hz); elemental analysis, calcd. for $C_{21}H_{27}NO_{10}$ (453.45): C=55.62, H=6.00, N=3.09; found: C=55.22, H=5.93, N=3.11.

EXAMPLE 15

Preparation of 15, Tetraethyl(4-aminobenzylidene)dimalonate:

A solution of tetraethyl(4-nitrobenzylidene)dimalonate 1.5 g of in 200 ml of ethanol was shaken with 0.5 g of 5% palladium on carbon under 50 psi of hydrogen for 28 hr. The catalyst was filtered off and the filtrate was concentrated and dried under vacuum (0.05 mm Hg) overnite. The residue (1.4 g) was homogeneous by TLC (silica gel; diethyl ether (20%):hexanes(80%). $^1$H-NMR (300MHz, $CDCl_3$) ∂ 1.1 (t, 6H, J = 7Hz), 1.3 (t, 6H, J = 7Hz), 3.7 (br s, 2H), 3.9-4.2 (complex mult. 7H), 6.6 (d, 2H, J = 9Hz), 7.2 (d, 2H, J - 9Hz).

EXAMPLE 16

Preparation of 16, 4-Benzyloxycarbonyl benzaldehyde:

To a solution of 4-carboxybenzaldehyde (15.02 g, 100 mmol) and triethylamine (14.1 ml, 101 mmol) in 250 ml dry methylene chloride (250 ml) cooled to 0°C in an ice bath was added benzyl chloroformate (14.3 ml, 100 mmol) followed by 4-dimethylaminopyridine (6.07 g, 50 mmol). The reaction mixture was stirred at 0°C for 3 hr, diluted with methylene chloride (250 ml), and washed with satd. NaCl (2x100 ml). Aqueous washes were extracted with methylene chloride (3x100 ml) and combined organics were washed with satd. $Na_2CO_3$ (3x100 ml) and satd. NaCl (1x100), dried over anhyd. $MgSO_4$ filtered, and concentrated to give 21.65 g yellow oil. The oil was dissolved in methylene chloride (20 ml) under nitrogen and pyridine (10 ml) and 4-dimethylaminopyridine (1.4 g) were added. The reaction mixture was heated under reflux for 2 hr, diluted with methylene chloride (200 ml), washed with 0.5N HCl (2x100 ml), satd. $NaHCO_3$ (2x100 ml), 0.5N HCl (2x100 ml), $H_2O$ (1x100 ml), and satd. NaCl (1x100 ml), dried over anhyd. $MgSO_4$, filtered, and concentrated to give 19.0 g orange oil. Crude product was purified by bulb-to-bulb distillation (170°C @ 0.3 torr) to yield 15.2 g (67%) 4-benzyloxycarbonyl benzaldehyde as a clear oil: $^1$H-NMR (300MHz, $CDCl_3$) $\delta$ 5.40 (s, 2H), 7.32-7.50 (complex mult., 5H), 7.95 (d, 2H, J = 8.1Hz), 8.23 (d, 2H, J = 8.1Hz), 10.10 (s, 1H).

EXAMPLE 17

Preparation of 17, Diethyl(4-benzyloxycarbonyl)benzylidene malonate:

A mixture of 4-benzyloxycarbonyl benzaldehyde (12.3 g, 51 mmol), diethyl malonate (9.3 ml, 61 mmol), piperidine (0.6 ml, 6 mmol), and benzene (25 ml) was heated under vigorous reflex with azeotropic removal of water for 26 hr. The reaction mixture was concentrated in vacuo to give a yellow oil which was dissolved in ethyl acetate (150 ml), washed with 1N HCl (2x100 ml), dil. $NaHCO_3$ (1x100 ml), and satd. NaCl (1x100 ml), dried over anhyd. $Na_2SO_4$, filtered, and concentrated to give 21.8 g light yellow oil. Crude product was purified by low pressure chromatography (silica gel 230-400 mesh; diethyl ether (35%):hexanes (65%) to yield 17.0 g (87%) diethyl (4-benzyloxycarbonyl)benzylidene malonate as a clear oil: $^1$H-NMR (300MHz, $CDCl_3$) $\delta$ 1.27 (t, 3H, J = 7Hz), 1.34 (t, 3H, J = 7Hz), 4.32 (q, 4H, J = 7Hz), 5.37 (s, 2H), 7.35-7.48 (complex mult., 5H), 7.50 (d, 2H, J = 8.6Hz), 7.74 (s, 1H) 8.07 (d, 2H, J = 8.6Hz); elemental analysis, calcd. for $C_{22}H_{22}O_6$ (382.41): C=69.10, H=5.80, N=0; found: C=68.98, H=5.89, N=0).

EXAMPLE 18

Preparation of 18, Tetraethyl(4-benzyloxycarbonyl)benzylidene malonate:

To a stirred suspension of sodium hydride (54 mmol, from 2.15 g 60% in oil dispersion, rinsed twice with dry hexanes) in 100 ml dry diethyl ether under nitrogen was added dropwise a solution of diethyl malonate (7.5 mol, 50 mmol) in 30 ml dry diethyl ether. The resultant thick suspension was stirred at RT for 30 min then a solution of diethyl (4-benzyloxycarbonyl) benzylidene malonate (15.72 g, 41 mmol) in 130 ml dry diethyl ether was added dropwise. The reaction mixture was stirred at RT for 1.5 hr then poured into 1N HCl (400 ml). The organic phase was collected and the aqueous phase was extracted with ethyl acetate (3x100 ml). Combined organics were washed with satd. NaCl (1x100 ml), dried over anhyd. $Na_2SO_4$, filtered, and concentrated to give 23.10 g light yellow oil. Crude product was purified by low pressure chromatography (silica gel 230-400 mesh; diethyl ether (40%):hexanes (60%) to give 19.93 g (85%) tetraethyl (4-benzyloxycarbonyl) benzylidene dimalonate as a clear oil: $^1$H-NMR (300MHz, $CDCl_3$) $\delta$ 1.03 (t, 6H, J = 7Hz), 1.22 (t, 6H, J = 7Hz), 3.94 (q, 4H, J = 7Hz), 4.10-4.31 (complex mult., 7H), 5.33 (s, 2H), 7.32-7.46 (complex mult., 5H), 7.44 (d, 2H, J = 8Hz), 7.96 (d, 2H, J = 8Hz) elemental analysis, calcd. for $C_{29}H_{34}O_{10}$ (542.58): C=64.20, H-6.32, N=-0; found: C=63.89, H=6.39, N=0.

EXAMPLE 19

Preparation of 19, Tetraethyl 4-carboxybenzylidene dimalonate:

A mixture of tetraethyl (4-benzyloxycarbonyl)benzylidene diamalonate (3.92 g, 7.2 mmol) and 5% palladium on carbon catalyst (0.51 g) in 50 ml abs. ethanol was shaken under $H_2$(50 psi) in a Parr® apparatus for 17 hr then filtered through a pad of Celite®. Filtrate was concentrated in vacuo to give 3.30 g (quant. yield) tetraethyl 4-carboxybenzylidene dimalonate as a white solid: $^1$H-NMR (300MHz, $CDCl_3$) ∂ 1.03 (t, 6H, J = 7Hz), 1.23 (t, 6H, J = 7Hz), 3.96 (q, 4H, J = 7Hz), 4.11-4.33 (complex mult., 7H), 7.48 (d, 2H, J = 8Hz), 8.03 (d, 2H, J = 8Hz).

## EXAMPLE 20

Preparation of 20, N-[Methyl-4-phenyl(tetraethyl-1,1,3,3-propane tetracarboxylate)-O-methyl-4-phenylsulfonyl(5-thiophene-2-sulfonamide)]carbamate:

To a stirred solution of 5-[(4-hydroxymethylphenyl)sulfonyl]thiophene-2-sulfonamide [Pfizer, Great Britain Patent #1,459,571 (Dec 22, 1976)] (1.010 g, 3.0 mmol) in 5.0 ml dry pyridine cooled in an ice bath and under nitrogen was added dropwise a solution of p-nitrophenyl chloroformate (0.619 g, 3.1 mmol) in 7 ml dry methylene chloride. The reaction mixture was stirred at 0° C for 4 hr and a solution of tetraethyl (4-aminomethylbenzylidene) dimalonate (2.88 g, 6.1 mmol) in 25 ml dry methylene chloride was added followed by N-methyl morpholine (0.67 ml, 6.1 mmol). The reaction mixture was allowed to warm to RT where it was stirred for 21 hr then concentrated in vacuo to give an orange oil. Oil was taken up in ethyl acetate (100 ml), washed with 0.5N HCl (3x20 ml), satd. $NA_2CO_3$ (12x20 ml), and satd. NaCl (1x50 ml), dried over anhyd. $MgSO_4$, filtered, and concentrated to give 2.1 g yellow glass. Crude product was purified by low pressure chromatography (silica gel, 230-400 mesh; ethyl acetate (50%):hexanes (50%) to yield 1.11 g (46%) 20 as a glassy solid: m.p. 57-61° C; $^1$H-NMR (300MHz, DMSO-$d_6$) ∂ 0.88 (t, 6H, J = 7.1Hz), 1.16 (t, 6H, J = 7.1Hz), 3.81 (q, 4H, J = 7.1Hz), 3.95-4.15 (complex mult., 7H), 4.14 (d, 2H, J = 5.6Hz), 5.13 (s, 2H), 7.12 (d, 2H, J = 8Hz), 7.24 (d, 2H, J = 8Hz), 7.59 (d, 1H, J = 4.2Hz), 7.61 (d, 2H, J = 8Hz), 7.87 (d, 1H, J = 4.2Hz), 7.92 (t, 1H, J = 5.6Hz), 8.00 (s, 2H), 8.04 (d, 2H, J = 8Hz); elemental analysis, calcd. for $C_{34}H_{40}N_2O_{14}S_3$ (796.88): C=51.25, H=5.06, N=3.52; found: C=51.43, H=5.32, N=3.39.

## EXAMPLE 21

Preparation of 21, 5-(3-Acetoxybenzoyl)thiophene-2-sulfonamide:

To a solution of 5-(3-hydroxybenzoyl)thiopnene-2-sulfonamide [Merck, U.S. Patent #4,486,444 (Dec. 4, 1984)] (283 mg, 1.0 mmol) in 5 ml dry acetone cooled to -10° C in an ice/methanol bath and under a drying tube was added triethylamine (153 µl, 1.1 mmol) followed by dropwise addition of acetyl chloride (95 µl) in 1 ml dry acetone. Reaction was stirred at -10° C for 4 hr then concentrated to dryness. Sample was purified by low pressure chromatography (silica gel, 230-400 mesh; ethyl acetate (50%):hexanes (50%) to give 281 mg (86%) 5-(3-acetoxybenzoyl)thiophene-2-sulfonamide as a white solid: $^1$H-NMR (300MHz, DMSO-$d_6$) ∂ 2.30 (s, 3H), 7.50 (d, 1H, J = 8Hz), 7.62-7.80 )(complex mult., 4H), 7.72 (d, 1H, J = 8Hz), 8.02 (s, 2H).

## EXAMPLE 22

Preparation of 22, N-[Carbonyl-4-benzene(tetraethyl-2-propane-1,1,3,3-tetracarboxylate]-5-(3-acetoxybenzoyl)thiophene-2-sulfonamide:

To a solution of the 5-(3-acetoxybenzoyl)thiophene-2-sulfonamide (240 mg, 0.74 mmol) and the acid chloride of tetraethyl 4-carboxy benzylidenedimalonate (freshly prepared from tetraethyl 4-carboxy-benzylidene dimalonate by reaction with oxalyl chloride in toluene) (662 mg, 0.91 mmol) in 15 ml dry ethyl acetate under nitrogen was added N-methyl morpholine (161 ul, 1.5 mmol). The reaction mixture was stirred at RT for 19 hr, N,N-dimethylaminopyridine (80 mg, 0.66 mmol) was added, and after 2 hr at RT the solution was concentrated in vacuo. Sample was purified by low pressure chromatography (silica gel, 230-400 mesh; ethyl acetate:hexanes gradient [50%:50% to 100%:0%]) twice to give 280 mg (42%) N-[carbonyl-4-benzene(tetraethyl-2-propane-1,1,3,3-tetracarboxylate]-5-(3-acetoxybenzoyl)thiophene-2-sulfonamide as a glass: $^1$H-NMR (300MHz, $CD_3CN$) ∂ 0.93 (t, 6H, J = 7.1Hz), 1.18 (t, 6H, J = 7.1Hz), 2.18 (s, 3H), 2.26 (s, 1H), 3.83 (q, 4H, J = 7.1Hz), 3.98-4.17 (complex mult., 7H), 7.30-7.72 (complex mult., 4H), 7.33 (d, 2H, J = 8.3Hz), 7.45 (d, 1H, J = 4.0Hz), 7.62 (d, 1H, J = 4.0Hz), 7.97 (d, 2H, J = 8.3Hz).

## EXAMPLE 23

Preparation of 23, N-[Carbonyl-4-benzene(tetralithio-2-propane-1,1,3,3-tetracarboxylate]-5-(3-Hydroxybenzoyl)thiophene-2-sulfonamide:

To a solution of the N-[carbonyl-4-benzene(tetraethyl-2-propane-1,1,3,3-tetracarboxylate]-5-(3-acetoxybenzoyl)thiophene-2-sulfonamide (21.06 mg, 0.028 mmol) in 1 ml abs. ethanol was added 1 ml water followed by anhyd. lithium hydroxide (8.6 mg, 0.36 mmol). Reaction mixture was heated at 60° C for 22 hr with stirring then was concentrated in vacuo. Residue was dissolved in water and the solution was adjusted to pH 8.5 by the addition of 1N HCl. The solution was filtered through a medium frit and the volume was adjusted to 4.0 ml with water. Reverse phase HPLC (SpectraPhysics system, Waters Bondapack C18 column, flow rate = 1ml/min, $H_2O:CH_3CN$ gradient [98%:2% to 50%:50%]) indicated purity to be 92% (@254 nm).

## EXAMPLE 24

Preparation of 24 N-[N-α-tert-butoxycarbonyl-L-leucyl]-5-(3-hydroxybenzoyl)thiophene-2-sulfonamide:

To a magnetically stirred mixture of 0.75 g (3 mmol) of N- -BOC-leucine monohydrate, 0.283 g (1 mmol) of 5-(3-hydroxybenzoyl)thiophene-2-sulfonamide [Merck, U.S. Patent #4,486,444 (Dec. 4, 1984)], 0.34 ml (3 mmol) of N-methylmorpholine, and 0.37 g (3 mmol) of 4-dimethylaminopyridine in 15 ml of ethyl acetate was added 620 mg (3 mmol) of 1,3-dicyclohexylcarbodiimide. The mixture was allowed to stir overnight at room temperature, acidified with 25 ml of 1M citric acid (aq), and filtered. The organic layer was separated and washed with 10 ml of 1M citric acid, then concentrated to dryness. The residue (2 spots by TLC; methanol (10%):chloroform (90%) was taken up in 10 ml of ethanol and basified to pH ~11 with 0.5 N NaOH. After 0.5 hr at room temperature, the mixture was acidified with 1M citric acid and extracted with 100 ml of ethyl acetate. The organic layer was washed with water (2x50 ml) and satd. NaCl, dried over anhyd. $MgSO_4$, filtered, and concentrated to dryness. The residue was purified by low pressure chromatography (silica gel, 230-400 mesh; ethyl acetate (50%):chloroform (50%), then methanol (5%):ethyl acetate (45%):chloroform (50%), and finally methanol (10%):ethyl acetate (40%):chloroform (50%) to give 0.51 g N-[N- -tert-butoxycarbonyl-L-leucyl]-5-(3-hydroxybenzoyl)thiophene-2-sulfonamide as a white foam. $^1$H NMR of the product (DMSO-$d_6$) was consistent with the desired structure.

## EXAMPLE 25

Preparation of 25, N-[L-leucyl]-5-(3-hydroxybenzoyl)thiophene-2-sulfonamide:

A solution of 150 mg of N-[N-tert-butoxycarbonyl-L-leucyl]-5-(3-hydroxybenzoyl)thiophene-2-sulfonamide in 20 ml of 5.12 N ethanolic HCl was stirred at room temperature for 5 hr then concentrated to

dryness. The residue was suspended in 200 ml of ethyl acetate and filtered. The cryrstalline solid was collected and dried at 0.5 mm Hg over $P_2O_5$ for 1 hr. Elemental analysis; calcd. for $C_{17}H_{20}N_2O_5S_2$ (396.49): C=51.50, H=5.08, N-7.06; found: C=51.73, H=5.29, N=6.70.

## EXAMPLE 26

Preparation of 26, N-[N-α-[carbonyl-4-benzene(tetraethyl-2-propane-1,1,3,3-tetracarboxylate)]-L-leucyl]-5-(3-hydroxybenzoyl)thiophene-2-sulfonamide:

A solution of 300 mg (0.58 mmol) of N-[L-leucyl]-5-(3-hydroxybenzoyl)thiophene-2-sulfonamide in 20 ml of 5.12 N ethanolic HCl was kept at room temperature for 12 hr, then concentrated to dryness. To a stirred suspension of the residue in 10 ml of ethyl acetate was added 0.2 ml (1.7 mmol) of N-methylmorpholine and 0.87 mmol of the acid chloride of tetraethyl 4-carboxy benzylidenedimalonate (freshly prepared from tetraethyl 4-carboxybenzylidene dimalonate by reaction with oxalyl chloride in toluene). Pyridine (5 ml) was added and the mixture was allowed to stir for 3 hr at room temperature. The mixture was diluted with 100 ml of ethyl acetate and washed with 0.1 N HCl (2x50 ml). The organic extracts were dried over anhyd. $MgSO_4$, filtered, and concentrated to dryness. Low pressure chromatography (silica gel; 230-400 mesh; 2" x 6" column; ethyl acetate (50%):methylene chloride (50%), then methanol (5%):ethyl acetate (45%)-:methylene chloride (50%) gave two products. The product which eluted first was the diacylated compound (320 mg). The second product eluted was the mono-acylated compound (260 mg) and was dried for 12 hr at 0.01 mm Hg over $P_2O_5$ @ 25° C. $^1$H-NMR (300MHz, DMSO-$d_6$) ∂ 0.85 (br t, 6H, J = 7.5Hz), 0.9 (t, 6H, J = 7.5Hz), 1.16 (t, 6H, J = 7.5Hz), 1.58 (br mult., 3H), 3.85 (q, 4H, J = 7.5Hz), 4.08 (complex mult., 8H), 4.35 (br mult., 1H), 7.05-7.75 (complex mult., 10H), 8.0 (br s, 1H), 9.8 (s, 1H); elemental analysis, calcd. for $C_{39}H_{46}N_2O_{14}S_2 \cdot 0.5\ H_2O$ (839.94): C=55.78, H=5.64, N=3.34; found: C=55.76, H=5.87, N=3.35.

## EXAMPLE 27

Preparation of 27, N-[N-α-[Carbonyl-4-benzene(tetralithio-2-propane-1,1,3,3-tetracarboxylate)]-L-leucyl]-5-(3-hydroxybenzoyl)thiophene-2-sulfonamide:

To a solution of 20 mg (0.024 mmol) N-[N- -[carbonyl-4-benzene(tetraethyl-2-propane-1,1,3,3-tetracarboxylate)]-L-leucyl]-5-(3-hydroxybenzoyl)thiophene-2-sulfonamide in 1.0 ml of ethanol was added 1.0 ml of water and 8 mg (0.34 mmol) of anhyd. lithium hydroxide. The mixture was warmed to 60° C with stirring for 12 hrs, then cooled to room temperature, and neutralized to pH 8.0-8.5 with 0.1N HCl. The solution was concentrated to dryness, dissolved in 2 ml of distilled water, filtered (1.5 ml water rinse), and diluted to 3.5 ml in a graduated centrifuge tube. Reverse phase HPLC (SpectraPhysics system, Waters Bondapack C18 column, flow rate - 1ml/min, $H_2O$:$CH_3CN$ gradient [98%:2% to 50%:50%]) indicated purity of solution to be 88.5% (@ 254 nm).

## EXAMPLE 28

Preparation of 28, Dibenzyl(4-cyanobenzylidene)malonate:

A mixture of 4-cyanobenzylaldehyde (25.0 g 191 mmol), dibenzyl malonate (58.0 g, 204 mmol) piperidine (3.0 ml, 30 mmol) and benzene (150 ml) was heated under vigorous reflux with azeotropic removal of water for 22 hours. The reaction mixture was concentrated in vacuo to give a yellow oil which was dissolved in ethyl acetate (300 ml), washed with 1 N HCl (1 x 100 ml) satd. $NaHCO_3$ (1 x 100 ml), and brine (1 x 100 ml), dried over anhyd. magnesium sulfate filtered, and concentrated to give a yellow oil. Crude product was recrystallized from diethyl ether to give a white solid which was collected by vacuum

filtration and air dried to yield dibenzyl(4-cyanobenzyledene)malonate (46.5 g, 61% yield) as a white solid: m.p. 80-81 °C; $^1$H-NMR (400MHz, $CDCl_6$) ∂: 5.28 (d, 4H, J = 75 Hz), 7.25-7.45 (complex mult, 14H) 7.74 (s, 1H).

Preparation of 29, Tetrabenzyl(4-cyanobenzylidene)dimalonate:

To a mechanically stirred suspension of sodium hydride (139 mmol, from 5.56 g 60% in oil dispersion, rinsed twice with dry hexanes) in 200 ml dry diethyl ether under nitrogen was added dropwise a solution of dibenzyl malonate (43.0 g, 151 mmol) in 100 ml dry diethyl ether. The resultant thick suspension was stirred at room temperature for 30 minutes than a solution of dibenzyl (4-cyanobenzylidene)malonate (46.0 g, 116 mmol) in 1.51 dry diethyl ether was added in a fine stream. The reaction mixture was stirred at 25 °C for 5 days then poured into 1N HCl (500 ml). The organic phase was collected and the aqueous phase was extrated with ethyl acetate (4 x 200 ml). The combined organics were washed with brine (1 x 200 ml), dried over anhydrous magnesium sulfate, filtered, and concentrated to give a yellow oily solid. Crude product was recrystallized from diethyl ether, collected by vacuum filtration, and air dried to give tetrabenzyl(4-cyanobenzylidene) dimalonate (70.7 g, 90% yield) as a fine white solid: m.p. 84-85.5 °C; $^1$H-NMR (300MHz, $CDCl_3$) ∂: 4.21 (s, 3H), 4.86 (s, 4H), 5.04 (d, 4H, J = 5.9Hz), 7.04 (d, 4H, J = 7.1Hz), 7.20-7.32 (complex mult., 20H), elemental analysis, calcd. for $C_{42}H_{35}NO_8$ (681.74): C=74.00, H=5.17, N=2.05; found: C=74.02, H=4.97, N=1.98.

Preparation of 30, Tetrabenzyl(4-aminomethylbenzylidene)dimalonate:

To a stirred suspension of sodium borohydride (5.25 g, 139 mmol) in 60 ml dry tetrahydrofuran under nitrogen was added dropwise a solution of trifluoroacetic acid (10.7 ml, 139 mmol) in 25 ml dry tetrahydrofuran over a period of 10 minutes. To the stirred white suspension was added over 1 hour a solution of tetrabenzyl (4-cyanobenzylidene)dimalonate (18.9 g, 27.7 mmol) in 120 ml dry tetrahydrofuran. After stirring at 25 °C for 5 hours, the reaction was chilled in an ice bath and quenched by the cautious addition of 100 ml water. After concentration under reduced pressure the residue was taken up in water (200 ml) and the solution was extracted with methylene chloride (3 x 100 ml); the combined organics were washed with water (3 x 50 ml) and brine (1 x 50 ml), dried over anhydrous magnesium sulfate, filtered and concentrated to yield the crude amine (18.21 g) as a clear oil.

To a stirred solution of the crude amine in chloroform (200 ml) was added di-tert-butyldicarbonate (27.4 g, 125 mmol). After 5 days at 25 °C the solution was concentrated and the resultant residue was purified by low pressure chromatography (silica gel, 230-400 mesh, 10 x 16 cm column; ethyl acetate (20%):hexanes (80%)) to yield tetrabenzyl-4-(N-(1,1-dimethylethoxycarbonyl)aminomethy lbenzylidene)dimalonate (10.3 g, 47% yield) as a clear oil: $^1$H-NMR (400MHz, $CDCl_3$) ∂ 1.47 (s, 9H), 4.11-4.24 (mult, 5H) 4.65 (br s, 1 H), 4.84 (s, 4H), 5.03 (d, 4H, J = 7.6Hz), 6.97 (d, 2H, J = 5.1Hz), 7.04 (d, 3H, J = 4.1Hz), 7.15 (d, 2H, J = 5.1Hz), 7.20-7.30 (complex mult., 17H), elemental analysis, calcd. for $C_{47}H_{47}NO_{10}$ (785.89): C=71.83, H=6.03, N=1.78; found: C=72.22, H=5.86, N=1.76.

A solution of tetrabenzyl-N-tert-butoxycarbonyl(4-aminomethylbenzylidene)dimalonate (3.03 g, 3.86 mmol) in 35 ml formic acid (96%) under nitrogen was stirred at 25 °C for 3.5 days then concentrated. The resultant oil was taken up in satd. $NaHCO_3$ (200 ml) and the solution was extracted with diethyl ether (3 x 150 ml); combined organics were washed with brine (1 x 100 ml), dried over anhydrous magnesium sulfate, filtered, and concentrated to yield tetrabenzyl(4-aminomethylbenzylidene)dimalonate (2.45 g) as a clear oil: $^1$H-NMR (300MHz, $CDCl_3$) ∂: 4.24 (mult, 3H) 4.5 (br s, 2H), 4.84 (2, 4H), 5.04 (d, 4H, J = 4.2Hz), 5.30 (s,2H), 7.06-7.30 (complex mult, 20H), 7.37 (d, 4H, J = 4.2Hz).

Preparation of 31, 3-O-Benzyl-estra-1,3,5(10)-triene-3,17β-diol:

To a stirred suspension of sodium hydride (23.2 mmol, from 0.928 g 60% dispersion in oil, rinsed thrice with dry hexanes) in 10 ml dry dimethylformamide cooled in an ice/water bath to 0 °C and under nitrogen was added in a steady stream a solution of estra-1,3,5(10-triene-3,17β-diol (4.30 g, 15.8 mmol). The reaction was allowed to warm to 25 °C where it was stirred for 1 hour. After addition of benzyl bromide (2.76 ml, 23.2 mmol) the reaction was stirred for 18 hours at 25 °C. The reaction mixture was concentrated under high vacuum, the residue was taken up in 10% citric acid (150 ml), and extracted with ethyl acetate (3 x

100 ml). The combined organics were washed with sat.d $NaHCO_3$ (1 x 150 ml) and brine (1 x 50 ml), dried over anhydrous magnesium sulfate, filtered, and concentrated to give a light tan oily solid. Solid was triturated with diethyl ether:hexanes to give 3-O-benzyl-estra-1,3,5(10)-triene3,17β-diol (1.80 g, 77% yield) as a fluffy white solid: m.p. 97-100 °C; $^1$H-NMR (300MHz, $CDCl_3$) ∂: 0.78 (s, 3H), 0.88 (t, 1H, J = 8Hz), 1.2-1.6 (complex mult, 2H), 1.65-1.75 (mult, 1H), 1.84-1.97 (complex mult, 2H), 2.05-2.20 (complex mult, 2H), 2.28-2.35 (mult, 1H), 2.82-2.85 (mult, 2H) 3.73 (t, 1H, J = 8.5Hz): 5.03 (s, 2H), 6.72 (d, 1H, J = 2.6Hz), 6.78 (dd, 1H, $J_1$ = 8.5Hz, $J_2$ = 2.6Hz), 7.20 (d, 2H, J = 8.5 Hz), 7.30-7.45 (complex mult, 5H); elemental analysis calcd. for $C_{25}H_{30}O_2$ (362.513): C=82.83, H=8.34, N=0; found: C=82.68, H=8.53, N=0.

Preparation of 32, 3-O-Benzyl-estra-1,3,5(10) triene-3,17β-diol-17 [N-(methyl-4-phenyl(tetrabenzyl-2-propane-1,1,3,3-tetracarboxylate))-carbamate]:

A stirred solution of 3-O-benzyl-estra-1,3,5(10)-triene-3,17β-diol (3.18 g, 8.77 mmol) in 75 ml dry benzene chilled in an ice/water bath to 0 °C was treated with phosgene (g) for 10 minutes. The solution was tightly stoppered and allowed to warm to 25 °C where it was stirred for 65 hours. Concentration of the solution gave a white solid which was dried under high vacuum. To a stirred solution of the solid in 25 ml dry benzene under nitrogen was added a solution of tetra benzyl (4-aminomethyl benzylidene malonate) amine (4.45 g, 6.49 mmol) in 50 ml dry benzene. After 30 minutes pyridine (0.78 ml, 9.64 mmol) was added. The reaction mixture was concentrated after stirring at 25 °C for 48 hours, the residue was taken up in 10% citric acid (300 ml), and the solution was extracted with ethyl acetate (3 x 200 ml). The combined organics were wahsed with 10% citric acid (3 x 100), satd. $NaHCO_3$ (3x100 mL), and brine (1x100 mL), dried over anhydrous magnesium sulfate, filtered, and concentrated to give a clear oil. The crude product was purified by low pressure chromatography (silica gel, 230-400 mesh, 10 x 16 cm column; acetonitrile (2%): methylene chloride (98%)) to yield 3-O-benzyl-estra-1,3,5(10)-triene-3,17β-diol 17-[N-(methyl-4-phenyl-(tetrabenzyl-2-propane-1,1,3,3-tetracarboxylate))-carbamate] (4.78 g, 68% yield) as a glassy solid: m.p. 57-61 °C; $^1$H-NMR (300MHz, $CDCl_3$) ∂: 0.81 (s, 3H), 1.25-1.75 (complex mult, 5H) 1.75-1.81 (complex mult, 1H), 1.81-1.96 (mult, 2H), 2.15-2.32 (mult, 3H), 2.84 (mult, 2H), 4.23 (mult, 5H), 4.68 (t, 1H, J = 8Hz), 4.76 (mult, 1H), 4.85 (s, 5H), 5.03 (s, 4H), 5.05 (s, 2H), 6.71 (d, 1H, J = 2.5Hz), 6.77 (dd, 1H, $J_1$ = 9.0Hz, $J_2$ = 2.5Hz), 6.99 (d, 2H, J= 8.1 Hz), 7.05 (dd, 4H, $J_1$ = 7.7Hz, $J_2$ = 1.6 Hz), 7.16 (d, 2H, J = 8.2 Hz), 7.20-7.45 (complex mult, 22H): elemental analysis calcd. for $C_{68}H_{67}NO_{11}$ (1074.282): C=76.03, H=6.29, N=1.30; found: C=76.19, H=6.08, N = 1.28.

Preparation of 33, Estra-1,3,5(10)-triene-3,17β-diol-17-[N-(methyl-4-phenyl(tetrasodium-2-propane-1,1,3,3-tetracarboxylate))-carbamate]:

A mixture of 3-O-benzyl-estra-1,3,5(10)-triene-3,17β-diol-17 [N-(methyl-4-phenyl(tetrabenzyl-2-propane-1,1,3,3-tetracarboxylate))-carbamate] (4.68 g, 4.36 mmol), 10% palladium on carbon catalyst (0.819 g), sodium bicarbonate (1.53 g, 18.2 mmol), water (40 ml), and dioxane (40 ml) was shaken under $H_2$ (50 psi) in a Parr® apparatus for 46 hours then filtered. Filtrate was concentrated to 10 ml and diluted with dioxane to give a white solid which upon collection and drying under high vacuum yielded estra-1,3,5(10)-triene-3,17β-diol-17-[N-(methyl-4-phenyl(tetrasodium-2-propane-1,1,3,3-tetracarboxylate))-carbamate] (2.83 g, 91% yield) as the trihydrate: m.p. >300 °C (darkens @ 230 °C); $^1$H-NMR (300MHz, $D_2O$) ∂: 0.89 (s, 3H), 1.3-1.6 (complex mult, 5H) 1b-2.0 (complex mult, 5H), 2.1-2.4 (complex mult, 4H), 2.88 (d, 2H, J = 3.5 Hz), 3.89 (s, 5H), 4.06 (d, 1H, J= 6.6 Hz), 4.38 (d, 2H, J = 6.5Hz), 4.66 (s, 1H, 6.76 (s, 1H), 6.80 (d, 1H, J = 7.8 Hz), 7.30 (d, 3H, J = 8.6 Hz), 7.41 (s, 2H); elemental analysis calcd. for $C_{33}H_{33}NNa_4O_{11}\bullet 3H_2O$ (765.631): C=51.77, H=5.13, N=1.83; found: C=51.64, H=5.43, N = 1.68; MS (FAB) m/e 1073 (M-1), 982,892,789,684.

## EXAMPLE 29

Preparation of Estra-6,7-dideuterio-1,3,5(10)-3,17β-diol-17-[N-(methyl-4-phenyl(tetrasodium-2-propane-1,1,3,3-tetracarboxylate))-carbamate]:

Preparation of 34, 3-O-Benzyl-estra-1,3,5(10),6-tetraene-3-hydroxy-17-one:

A stirred mixture of estra-1,3,5(10),6-tetraene-3-hydroxy-17-one (1.00 g, 3.73 mmol) benzyl chloride (1.5 ml, 13 mmol), and potassium carbonate (2.4 g, 17.4 mmol) in 95% ethanol (90 ml) was heated at reflux under nitrogen for 5 hours, diluted with water, and cooled in an ice/water bath to give a white precipitate. Solid was collected by vacuum filtration air dired, and recrystallized from acetone:petroleum ether to give 3-O-benzyl-estra-1,3,5(10),6-tetraene-3-hydroxy-17-one (1.32 g, 99% yield) as a white solid: m.p. 152-153 °C; $^1$H-NMR (300MHz, $CDCl_3$) δ: 0.88 (s, 3H), 1.38-1.58 (mult, 2H) 1.63-1.83 (complex mult, 2H), 1.94-2.02 (mult, 1H), 2.10-2.62 (complex mult, 6H), 5.05 (s, 2H), 6.07 (d, 1H, J = 9.5 Hz), 6.51 (dd, 1H, $J_1$ = 9.5 Hz, $J_2$ = 2.6Hz), 6.75 (d, 1H, J = 2.7 Hz), 6.80 (dd, 1H, $J_1$ = 8.4 Hz, $J_2$ = 2.7 Hz) 7.16 (d, 1H, J = 8.3 Hz), 7.28-7.45 (complex mult 5H); elemental analysis, calc. for $C_{25}H_{26}O_2$ (358.48) C = 83.76, it = 7.31, N = O; Found: C = 83.92, H = 7.39, N = 0.0.

Preparation of 35, 3-O-Benzyl-estra-1,3,5(10),6-tetraene-3-hydroxy-17-diol:

To a stirred solution of 3-O-benzyl-estra-1,3,5(10),6-tetraene-3-hydroxy-17-one (1.06 g, 2.96 g), in dry methanol (250 ml) was added portionwise sodium borohydride (0.76 g, 20.1 mmol). After heating at 40 °C for 45 minutes the reaction was quenched by the addition of glacial acetic acid (20 ml) and poured into water (300 ml) to give a white precipitate. Solid was collected by vacuum filtration, air dried, and recrystallized from acetone:petroleum ether to give 3-O-benzyl-ester-1,3,5(10),6-tetraene-3,-17β-diol (1.0 g, 94% yield) as a white solid: m.p. 96-99 °C; $^1$H-NMR (300MHz, $CDCl_3$) δ: 0.78 (s, 3H), 1.26 (mult, 1H) 1.40 (d, 2H, J = 5.7 Hz), 1.37-1.58 (complex mult, 2H), 2.15 (s, 4H), 2.34 (m, 1H), 3.76 (q, 1H, J = 6.4 Hz), 5.06 (s, 2H), 5.97 (d, 1H, J = 9.6 Hz), 6.44 (dd, 1H, $J_1$ = 9.5 Hz $J_2$ = 2.6 Hz) 6.73 (d, 1H, J = 2.7 Hz), 6.80 (dd, 1H, $J_1$ = 8.2 Hz, $J_2$ = 2.7 Hz) 7.16 (d, 1H, J = 8.1 Hz), 7.28-7.45 (complex mult, 5H).

Preparation of 36, 3-O-Benzyl-estra-1,3,5(10) 6-tetraene-3,17β-diol-17[N-methyl-4-phenyl(tetrabenzyl-2-propane-1,1,3,3-tetracarboxylate))-carbamate]:

A stirred solution of 3-O-benzyl-estra-1,3,5(10)-6-estraene-3,17β-diol (1.0 g, 2.77 mmol) in 50 ml dry benzene chilled in an ice/water bath to 0 °C was treated with phosgene (g) for 10 minutes. The solution was tightly stoppered and allowed to warm to 25 °C where it was stirred for 42 hours. Concentration of the solution gave a white solid which was dried under high vacuum. To a stirred solution of the solid in 10 ml dry benzene was added a solution of tetrabenzyl (4-aminomethylbenzylidene) dimalonate (2.45 g, 3.58 mmol) in 10 ml dry benzene. After 15 minutes pyridine (0.314 ml, 3.98 mmol) was added. After stirring at 25 °C for 18 hours the reaction mixture was concentrated, the residue was taken up in 10% citric acid (300 ml), and the solution was extracted with ethyl acetate (3 x 200 ml). The combined organics were washed with 10% citric acid (3 x 100 ml), satd. $NaHCO_3$ (3 x 100 ml), and brine (1 x 100 ml), dried over anhydrous magnesium sulfate, filtered and concentrated to give 3.67 g clear oil. The crude product was purified by low pressure chromatography (silica gel, 230-400 mesh, 10 x 16 cm column; acetonitrile (2%): methylene chloride (98%)) to yield 3-O-benzyl-estra-1,3,5(10),6-tetraene-3,17β-diol-17-[N-(methyl-4-phenyl-(tetrabenzyl-2-propane-1,1,3,3-tetracarboxylate))-carbamate] (1.78 g, 60% yield) as a white foam; m.p. 53-56 °C; $^1$H-NMR (300MHz, $CDCl_3$) δ: 0.82 (s, 3H), 1.35-1.75 (complex mult, 6H) 1.83-1.97 (complex mult, 2H), 2.10-2.42 (complex mult, 4H), 4.23 (m, 5H), 4.71 (t, 1H, J = 8.3 Hz), 4.77 (mult, 1H), 4.85 (s, 5H), 5.03 (s, 2H), 5.05 (s, 4H), 5.98 (d, 1H, J = 9.3 Hz), 6.45 (dd, 1H, $J_1$ = 9.5 Hz, $J_2$ = 2.5 Hz), 6.73 (d, 1H, J = 2.5 Hz), 6.80 (dd, 1H, $J_1$ = 8.4 Hz, $J_2$ = 2.3 Hz) 7.00 (d, 2H, J = 8.0 Hz), 7.05 (dd, 4H, $J_1$ = 7.3 Hz, $J_2$ = 1.7 Hz) 7.16 (d, 2H, J = 8.0 Hz), 7.04-7.44 (complex mult, 22H); elemental analysis, calculated for $C_{68}H_{65}NO_{11}$ (1072.266): C=76.17, H=6.11, N=1.31; found: C=76.22, H=6.16, N= 1.29.

Preparation of 37, 3-O-Benzyl-estra-6,7-dideuterio-1,3,5(10)-triene-3,-17β-diol 17-[N-(methyl-4-phenyl-(tetra-sodium-2-propane-1,1,3,3-tetracarboxylate))-carbamate]:

A mixture of 3-O-benzyl-estra-1,3,5(10), 6-tetraene-3,17β-diol-17-[N-(methyl-4-phenyl(tetrabenzyl-2-propane-1,1,3,3-tetracarboxylate)) (20.5 mg, 0.019 mmol) and 5% platinum on carbon catalyst (9 mg) in dioxane (0.5 ml) was stirred under deuterium (at atmospheric pressure) for 30 minutes. To the reaction was added 10% palladium on carbon catalyst (16 mg), aqueous 0.5 N $NaHCO_3$ (0.153 ml), and water (1 ml).

After stirring under hydrogen at atmospheric pressure for 15 hours, the mixture was filtered through a pad of glass fiber; filtrate was concentrated to give 3-O-benzyl-estra-6,7-dideuterio-1,3,5(10)-triene-3, 17β-diol-17-[N-(methyl-4-phenyl-(tetrasodium-2-propane-1,1,3,3-tetracarboxylate))-carbamate] (10 mg, 73% yield) as a white glass: m.p. >300 °C (darkens @230 °C); $^1$H-NMR (300MHz, $D_2O$) ∂: 0.94 (s, 3H), 1.35-1.64 (complex mult, 5H) 1.64-1.94 (complex mult, 3H), 1.94-2.07 (complex mult, 2H), 2.21-2.30 (complex mult, 2H), 2.36-2.47 (complex mult, 1H), 2.11 (d, 1H, J = 3.4 Hz), 3.78 (d, 2H, J = 8.7 Hz), 3.98 (d, 1H, J = 8.5 Hz), 4.39 (s, 2H), 4.70 (t, 1H, J = 8.5 Hz), 6.79 (d, 1H, J = 2.1 Hz), 6.84 (dd, 1H, $J_1$ = 8.4 Hz, $J_2$ = 2.4 Hz), 7.18 (d, 2H, J = 8.0 Hz), 7.39 (d, 1H, J = 8.5 Hz), 7.49 (d, 2H, J = 8.0 Hz); MS (FAB) m/e 1075 (M-1), 984, 894, 684, 594.

## EXAMPLE 30

Preparation of 38, Estra-1,3,5(10)-triene-3,17β-diol-3-[N-(methyl-4-phenyl(tetrabenzyl-2-propane-1,1,3,3-tetracarboxylate))-carbamate]:

To a stirred solution of estra-1,3,5(10)-triene-3,17β-diol (1.38 g, 5.1 mmol) in 40 ml dry tetrahydrofuran cooled to 0 °C in an ice/water bath and under nitrogen was added dropwise a solution of n-butyl lithium in hexanes (3.5 ml, 1.6 M, 5.6 mmol) such that the temperature of the reaction remained below 1 °C. After 60 minutes the fine white suspension was added dropwise to a stirred solution of 4-nitrophenylchloroformate (1.13 g, 5.6 mmol) in 50 ml dry tetrahydrofuran cooled in an ice/methanol bath and under nitrogen such that the temperature of the reaction remained below -15 °C. After 30 minutes at -15 °C a solution of tetrabenzyl (4-aminomethylbenzylidene) dimalonate (3.52 g, 5.13 mmol) and diisopropylethylamine (1.77 ml, 10.2 mmol) in 100 ml dry tetrahydrofuran was added dropwise to the light green reaction mixture such that the temperature remained below -10 °C. The reaction mixture was stirred at -2 °C for 60 minutes, allowed to warm to 25 °C, and then concentrated under reduced pressure. The residue was taken up in satd. $NaHCO_3$ (100 ml) and extracted with diethyl ether (3 x 150 ml). Combined organics were washed with 10% citric acid (3 x 100 ml), satd $NaHCO_3$ (5 x 20 ml), and brine (1 x 100 ml), dried over anhydrous magnesium sulfate, filtered and concentrated to give a oily solid. The crude product was purified by low pressure chromatography (silica gel, 230-400 mesh, 8 x 16 cm column; acetonitrile (5%): methylene chloride (95%) to yield estra-1,3,5(10)-triene-3,17β-diol-3[N-(methyl-4-phenyl(tetrabenzyl-2-propane-1,1,3,3-tetracarboxylate))-carbamate] (2.37 g, 48% yield) as a clear glass: m.p. 57-61 °C; $^1$H-NMR (300MHz, $CDCl_3$) ∂: 0.77 (s, 3H), 1.18-1.54 (complex mult, 6H) 1.65-1.72 (mult, 1H), 1.85-1.97 (complex mult, 2H), 2.09-2.85 (complex mult, 3H), 2.84 (d, 2H, J = 3.4 Hz), 3.72 (dd, 1H, $J_1$ = 5.6 Hz, $J_2$ = 2.1 Hz), 4.24 (s, 3H), 4.32 (d, 2H, J = 5.9 Hz), 4.85 (s, 4H), 5.04 (d, 4H, J = 5.7 Hz), 5.06 (mult, 1H), 6.86 (s, 1H), 6.89 (d, 1H, J = 8.3 Hz), 7.01-7.07 (mult, 6H), 7.17-7.31 (mult, 19H): elemental anaylsis, calcd. for $C_{61}H_{59}NO_{11}$ (982.14): C=74.60, H=6.06, N= 1.43; found: C=74.32, H=6.32, N=1.45.

Preparation of 39, Estra-1,3,5(10)-triene-3,17β-diol-3-[N-(methyl-4-phenyl(tetrasodium-2-propane-1,1,3,3-tetracarboxylate))carbamate]:

A mixture of estra-1,3,5(10)-triene-3,17β-diol-3-[N-(methyl-4-phenyl(tetrabenzyl-2-propane-1,1,3,3-tetracarboxylate))carbamate] (5.83 mg, 0.593 mmol), 10% palladium on carbon catalyst (135 mg), sodium bicarbonate (204 mg, 2.43 mmol), water (10 ml), and dioxane (10 ml) was shaken under $H_2$ (50 psi) in a Parr® apparatus for 17 hours then filtered. Filtrate was concentrated to 2 ml and diluted with dioxane to give a white solid which upon collection and drying under high vacuum yielded estra-1,3,5(10)-triene-3,17β-diol-3-[N-(methyl-4-phenyl(tetrasodium-2-propane-1,1,3,3-tetracarboxylate))carbamate] (242 mg, 50% yield) as the trihydrate/dioxane solvate: m.p. >300 °C (darkens @ 230 °C); $^1$H-NMR (300MHz, $D_2O$) ∂: 0.76 (s, 3H), 1.23-1.50 (complex mult, 6H) 1.65-1.80 (mult, 1H), 1.91 (d, 2H, J = 11 Hz), 2.05-2.12 (mult, 1H), 2.20-2.30 (mult, 1H), 2.33 (d, 1H, J = 11 Hz), 2.85 (br s, 2H), 3.67 (d, 2H, J = 7.8 Hz), 3.76 (s, 4H), 3.86 (d, 1H, J = 7.1 Hz), 4.34 (s, 2H), 6.92 (s, 1H), 6.94 (d, 1H, J = 10 Hz), 7.21 (d, 2H, J = 7.0 Hz), 7.38 (d, 2H, J = 7.0 Hz), 7.40 (d, 1H, J = 10 Hz); elemental anaylsis, calcd. for $C_{33}H_{33}NNa_4O_{11}•3H_2O•$ $1/2C_4H_8O_2$ (809.68): C=51.92, H=5.35, N= 1.73; found: C=51.92, H=5.72, N= 1.72.

## Claims

1. A compound of the formula:

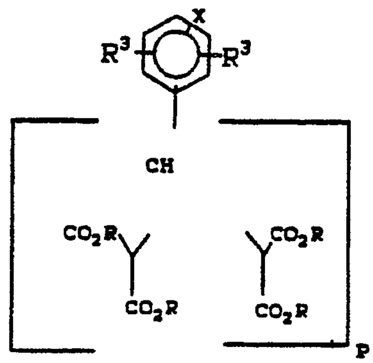

wherein:
X is
(1) hydrogen;
(2) alkyl having 1 to 6 carbon atoms
(e) $-(CH_2)_n\ (R^2)_2$ M
wherein n is 0 to 6 and M is
a) halogen;
b) $-NR^1R^1$;
c) $-NHC(O)R^1$;
d) $-NO_2$;
e) $-C(O)R^1$;
f) $-C(O)OR^1$;
g) -CN;
each $R^1$ is independently H, $C_1$ to $C_6$ straight or branched alkyl, $(CH_2)_m$ OH where m is 1 to 3, benzyl or substituted benzyl wherein the substituents are $C_1$ to $C_6$ alkyl, halogen, CN or $CF_3$, phenyl or substituted phenyl wherein the substituents are $C_1$ to $C_6$ alkyl, halogen, CN or $CF_3$;
$R^2$ is about or is H, $C_1$ to $C_6$ straight or branched alkyl, $C_3$ to $C_6$ cycloalkyl, $(CH_2)_m$ OH where m is 1 to 3;
$R^3$ is H, $C_1$ to $C_6$ straight or branched alkyl, $(CH_2)_m$ OH where m is 1 to 3;
R is H, $C_1$ to $C_6$ alkyl, benzyl or a pharmaceutically acceptable salt; and
p is 1 or 2.

2. A compound according to Claim 1 which is:
tetraethyl(4-cyanobenzylidene)dimalonate;
tetraethyl(4-aminomethylbenzylidene)dimalonate;
tetraethyl-N-benzoyl(4-aminomethylbenzylidene)dimalonate;
tetraethyl-N-4-iodobenzoyl(4-aminomethylbenzylidene)dimalonate;
tetralithium-N-benzoyl(4-aminomethylbenzylidene)dimalonate;
tetralithium-N-4-iodobenzoyl(4-aminomethylbenzylidene)dimalonate;
1,3-bis[tetraethyl(2-propane-1,1,3,3-tetracarboxylate)]-5-nitrobenzene;
3,5-bis[tetraethyl(2-propane-1,1,3,3-tetracarboxylate)]aniline;
tetraethyl(4-nitrobenzylidene)dimalonate;
tetraethyl(4-aminobenzylidene)dimalonate;
tetraethyl(4-benzyloxycarbonyl)benzylidene dimalonate;
tetraethyl 4-carboxybenzylidene dimalonate.
tetrabenzyl(4-cyanobenzylidene)dimalonate;
tetrabenzyl(4-aminomethylbenzylidene)dimalonate.

3. A compound of the formula:

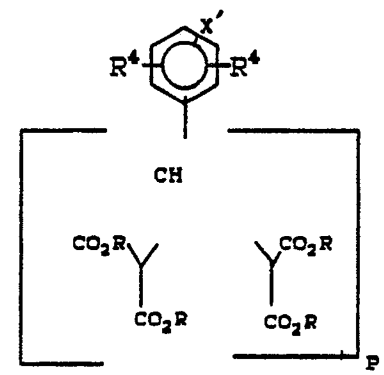

wherein:
X' is $-(CH_2)_n (R^5)_2$ M
wherein n is 0 to 6 and M is
a) $-NHC(O)R^7$;
b) $-OC(O)R^7$;
c)

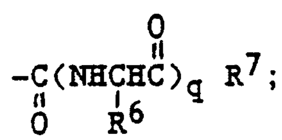

R is H, $C_1$ to $C_6$ alkyl, benzyl or a pharmaceutically acceptable salt;
p is 1 or 2;
q is 0 or 3;
$R^4$ is H, $C_1$ to $C_6$ straight or branched alkyl, $(CH_2)_m$ OH where m is 1 to 3;
$R^5$ is absent or is H, $C_1$ to $C_6$ straight or branched alkyl, $C_3$ to $C_6$ cycloalkyl, $(CH_2)_m$ OH where m is 1 to 3;
$R^6$ is a natural amino acid side chain; and
$R^7$ is a deprotonated bone resorption inhibiting or bone formation stimulating agent.

4. A compound according to Claim 3, wherein:
X' is $C_1$ to $C_6$ alkylamine;
R is H, $C_1$ to $C_6$ alkyl, benzyl or a pharmaceutical acceptable salt;
$R^7$ is a carbonic anhydrase inhibitor or estrogenic compound .

5. A compound of Claim 3, which is:
N-[methyl-4-phenyl(tetraethyl-1,1,3,3-propane tetracarboxylate)-0-methyl-4-phenyl-sulfonyl(5-thiophene-2-sulfonamide)]-carbamate;
N-[carbonyl-4-benzene(tetraethyl-2-propane-1,1,3,3-tetracarboxylate]-5-(3-acetoxybenzoyl)thiophene-2-sulfonamide;
N-[carbonyl-4-benzene(tetralithio-2-propane-1,1,3,3-tetracarboxylate]-5-(3-hydroxybenzoyl)thiophene-2-sulfonamide;
N-[N-α-[carbonyl-4-benzene(tetraethyl-2-propane-1,1,3,3-tetracarboxylate)]-L-leucyl-5-(3-hydroxybenzoyl)-thiophene-2-sulfonamide;
N-[N-α-[carbonyl-4-benzene(tetralithio-2-propane-1,1,3,3-tetracarboxylate)]-L-leucyl]-5-(3-hydroxybenzoyl)-thiophene-2-sulfonamide;
Estra-1,3,5(10)-triene-3,17-beta-diol-17-[N-(methyl-4-phenyl(tetrasodium-2-propane-1,1,3,3-tetracarboxylate))-carbamate];
Estra-6,7-dideuterio-1,3,5(10)-triene-3,17-beta-diol-17-[N-(methyl-4-phenyl(tetrasodium-2-propane-1,1,3,3-tetracarboxylate))-carbamate];
Estra-1,3,5(10)-triene-3,17-beta-diol-3-[N-(methyl-4-phenyl(tetrasodium-2-propane-1,1,3,3-tetracarboxylate))-carbamate].

6. A pharmaceutical composition which comprises a compound according to Claim 1 and a pharmaceutically acceptable carrier.

7. A pharmaceutical composition which comprises, a compound according to Claim 3 and a pharmaceutically acceptable carrier.

8. The use of a compound of Claim 1 for the preparation of a medicament useful for treating bone diseases.

9. The use of a compound of Claim 3 for the preparation of a medicament useful for treating bone diseases.